(19) **Europäisches Patentamt / European Patent Office / Office européen des brevets**

(11) **EP 4 696 771 A1**

(12) **EUROPEAN PATENT APPLICATION**
published in accordance with Art. 153(4) EPC

(43) Date of publication:
**18.02.2026   Bulletin 2026/08**

(21) Application number: **23933158.0**

(22) Date of filing: **09.08.2023**

(51) International Patent Classification (IPC):
**C12N 5/0775** (2010.01)     **A61K 35/28** (2015.01)
**A61P 11/00** (2006.01)

(52) Cooperative Patent Classification (CPC):
**A61K 35/28; A61P 11/00; C12N 5/06**

(86) International application number:
**PCT/KR2023/011786**

(87) International publication number:
**WO 2024/214872 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2023   KR 20230048433**

(71) Applicant: **Portrai Inc.**
**Seoul 03136 (KR)**

(72) Inventors:
• **PARK, Jeong Bin**
  **Suwon-si Gyeonggi-do 16229 (KR)**
• **LEE, Jae Eun**
  **Seoul 03136 (KR)**
• **LEE, Dae Seung**
  **Seoul 04702 (KR)**
• **LEE, Dong Joo**
  **Seoul 03088 (KR)**

(74) Representative: **Cabinet Beau de Loménie**
**103, rue de Grenelle / CS 90800**
**75340 Paris Cedex 07 (FR)**

(54) **METHOD AND DEVICE FOR EXPLORING DISTRIBUTION, EFFICACY, ACTION, OR PHYSIOLOGICAL ACTIVITY OF GENOME-CONTAINING SUBSTANCES IN TISSUES OF INTEREST**

(57)   The present disclosure relates to a method and apparatus for exploring distribution, efficacy, action, or physiological activity of a genome-containing material in a tissue of interest.

FIG. 1

**Description**

[Technical Field]

The present disclosure relates to a method and apparatus for exploring distribution, efficacy, action, or physiological activity of a genome-containing material in a tissue of interest.

[Background Art]

Cell therapy involves introducing therapeutic cells into a patient and has attracted significant attention as a promising treatment option for various diseases. Recently, cell therapy has attracted attention as a promising treatment for various diseases, including cancer (1), autoimmune diseases (2), inflammatory diseases (3), and neurodegenerative diseases (4). A cell therapeutic agent is characterized by its ability to design cell functions beyond a blood-brain barrier (BBB) (5), biocompatibility, and applicability, which enhance the potential of cell therapy. With the advent of advanced techniques for cell manipulation (e.g., chimeric antigen receptor-T cell or NK cell therapy (CAR-T/NK), stem cells, tumor-infiltrating lymphocytes, or microbiome preparations), the potential of cell therapy continues to grow as a game-changing therapeutic approach.

Despite the potential of the cell therapy as described above, the cell therapy faces limitations in reproducibility of drug efficacy due to the nature of living cells, as well as faces significant limitations due to difficulties in preparation, delivery, and administration [6].

Traditional methods for evaluating the efficacy of the cell therapy often lack the resolution and accuracy necessary to provide meaningful insights into the complex interactions and molecular changes that occur during treatment. For example, polymerase chain reaction (PCR) is fundamentally performed on dissected organs, and limits the identification of microscopic characteristics of cell therapies, particularly the identification of the distribution of administered cells within tissues [7]. Fluorescently labeled cells, commonly used to image administered cells, have the drawback that a tracer is readily detached from the cells, limiting cell tracking [8]. Above all, it is important to note that this approach does not provide a comprehensive understanding of the detailed molecular changes and interactions occurring between administered cells and host cells within the target tissue. Therefore, there is an urgent need for innovative and comprehensive approaches to better understand the true potential of a cell therapeutic agent.

Spatially analyzed transcriptomes have emerged as a groundbreaking tool in molecular biology [9, 10]. By combining spatial information with gene expression data, this technology enables researchers to study dynamic changes in gene expression patterns within tissues and cells at an unprecedented level of detail. The spatially analyzed transcriptomes have the potential to innovate the evaluation of the cell therapeutic agent by providing a more comprehensive understanding of their impact at the cellular and molecular levels.

In particular, for the cell therapy, pharmacokinetics (PK) data is becoming an important validation parameter. For example, stem cell therapeutic agents may be used to treat liver fibrosis. In this case, the distribution of the stem cells within tissues is a crucial metric for evaluating the efficacy and safety of these therapies, which is considered highly important in the pharmaceutical industry, as it is also required for IND (Investigational New Drug) applications.

[Disclosure of Invention]

[Solution to Problem]

Accordingly, the present disclosure provides an exploration method and an exploration apparatus capable of providing accurate positional information of a cell therapeutic agent within a tissue to provide various information such as on which cell types are mainly distributed and how they are distributed after administration of the cell therapeutic agent, and further, what therapeutic mechanism the cell therapeutic agent has.

The present disclosure provides a method and apparatus useful for evaluating various types of characteristics, including pharmacokinetics, an action mode, etc., of a cell therapeutic agent at a tissue level, by exploring microscopic spatial distribution and underlying mechanisms of treatment of a cell therapeutic agent administered to a tissue of interest using spatially analyzed transcriptome (ST) information.

[Technical Solution]

According to an aspect in the present disclosure,

there is provided a method for exploring distribution, efficacy, action, or physiological activity of a genome-containing material within a tissue of interest, including:

obtaining spatially resolved transcriptome information of the tissue of interest by providing a genome-containing material of part or all of a genome not present in the tissue of interest to the tissue of interest that has or is induced by a disease or illness;

classifying tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs; and

exploring the distribution, efficacy, action, or physiological activity of the genome-containing material within the tissue of interest from the classified information.

According to another aspect in the present disclosure,
there is provided a method for classifying genome-derived transcriptome information from spatially resolved transcriptome information, including:

obtaining spatially resolved transcriptome information of the tissue of interest by providing a genome-containing material of part or all of a genome not present in the tissue of interest to the tissue of interest that has or is induced by a disease or illness; and

classifying genome-derived transcriptome information among spatially resolved transcriptome information using an integrated reference transcriptome library including first organism reference transcriptome information to which the tissue of interest belongs and second organism reference transcriptome information to which the genome or a genome-containing material belongs.

According to still another aspect in the present disclosure,

there is provided an apparatus (100) for exploring distribution, efficacy, action, or physiological activity of a genome-containing material in a tissue of interest, which includes

an information receiving unit (110) that obtains spatially resolved transcriptome information of the tissue of interest by providing a genome-containing material of part or all of a genome not present in the tissue of interest to the tissue of interest that has or is induced by a disease or illness, and receives the obtained information;

an origin exploration unit (120) that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs; and

a molecular marker exploration unit (130) that explores a molecular marker related to the distribution, efficacy, action, or physiological activity of the genome-containing material within the tissue of interest from the information of the origin exploration unit (120).

According to still yet another aspect in the present disclosure,

there is provided an apparatus (200) for classifying genome-derived transcriptome information from spatially resolved transcriptome information, including: an information receiving unit (110) that receives spatially resolved transcriptome information of the tissue of interest by providing a genome-containing material of part or all of a genome not present in the tissue of interest to the tissue of interest that has or is induced by a disease or illness; and

an origin exploration unit (120) that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs.

[Advantageous Effects]

According to the present disclosure, by using an integrated reference transcriptome library that includes first organism reference transcriptome information and second organism reference transcriptome information to which a genome or a genome-containing material belongs, it is possible to improve the accuracy of mapping and classification since a sequence is mapped to a reference transcriptome with a higher matching rate among the two reference transcriptomes during sequence mapping compared to using the first organism reference transcriptome information and the second organism reference transcriptome information, respectively.

According to the present disclosure, by providing the accurate positional information of the genome-containing material (e.g., the cell therapeutic agent) within the tissue, it is possible to provide various information such as on which cell types are mainly distributed and how they are distributed after administration of the material, and further, what therapeutic mechanism the material has in the tissue or possible side effects.

The present disclosure uses the spatially analyzed transcriptome (ST) information to explore the microscopic spatial distribution and fundamental mechanisms of treatment of the genome-containing materials (e.g., cell therapeutic agents) administered to the tissue of interest, which is useful for evaluating various characteristics, including the pharmacokinetics and action mode, etc., of the genome-containing materials at the tissue level.

Accordingly, compared to the conventional methods (e.g., PCR) that require significant time and expense to explore the distribution and effects of the genome-containing materials, this approach allows for a highly accurate and simple, cost-effective method to determine the distribution of the genome-containing material.

[ Brief Description of Drawings]

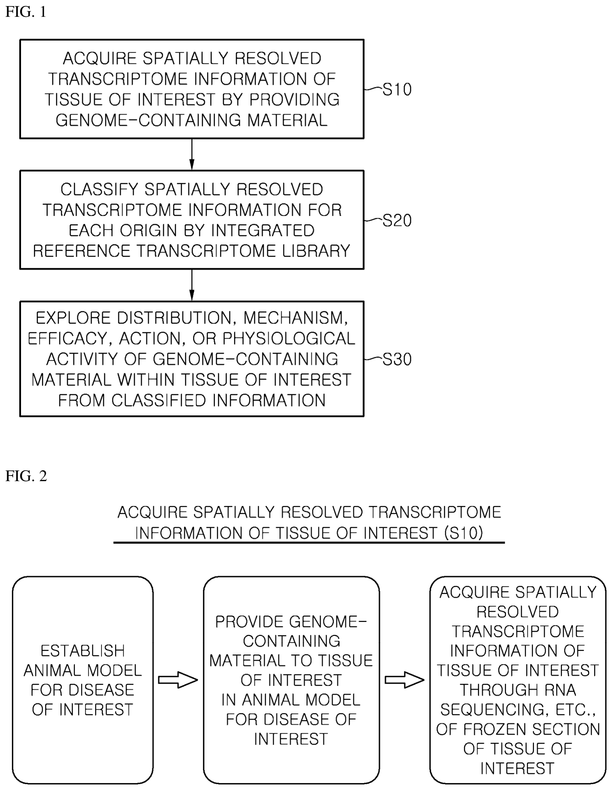

FIG. 1 is a flowchart of an exploration method according to an embodiment of the present disclosure.

FIG. 2 is a diagram illustrating a method for implementing a step (S10) of acquiring spatially resolved transcriptome information that shares spatial information of a tissue of interest having genome-containing material, according to an embodiment of the present disclosure.

FIG. 3 is a diagram illustrating the spatially resolved transcriptome information that shares spatial information of the tissue of interest, in step (S10) of acquiring the spatially resolved transcriptome information according to an embodiment of the present disclosure.

FIG. 4 is a diagram illustrating the results of constructing an integrated reference for a tissue containing gene-modified genome-containing material, despite having the same origin, and demonstrating an expression of a novel MUC1 isoform gene (denoted as MUCX), which is absent in the original reference.

FIG. 5 is a diagram illustrating an example of an analysis method or algorithm that may be used to explore the distribution, efficacy, action, or physiological activity of genome-containing material within a tissue of interest in exploration step (S30) according to an embodiment of the present disclosure, in which the well-known analysis method or algorithm such as SPADE, DEG, Stopover, CellDART, MIA, RCTD, and gene ontology analysis may be used.

FIG. 6 is a schematic diagram of a process for obtaining the spatially resolved transcriptome information of the tissue of interest in 'Nor', 'Con', and 'Exp' according to one implementation example of the present disclosure.

FIG. 7 is a diagram illustrating a normalized or non-normalized histogram of 'all_transcripts', 'all_human_transcripts', and '%human' in the 'Nor', 'Con', and 'Exp' according to one implementation example of the present disclosure.

FIG. 8 is a diagram illustrating a "spots_with_human_cell" variable in the "Nor," "Con," and "Exp" according to one implementation example of the present disclosure, which is a binary indicator that is yellow only when the %human in a spot exceeds a threshold.

FIG. 9 is a diagram illustrating an H&E stained image of the tissue of interest in three samples, "Nor," "Con," and "Exp," according to one implementation example of the present disclosure.

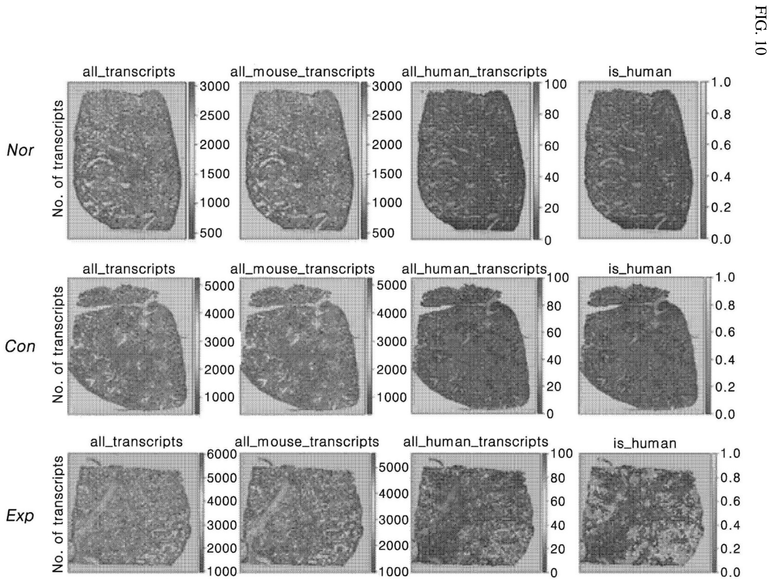

FIG. 10 is an image showing the total number (first column) of transcriptomes, the number (second column) of mouse transcriptomes, and the number (third column) of human transcriptomes in each of the three samples, "Nor," "Con," and "Exp," according to one implementation example of the present disclosure. Here, the human transcriptome is represented on a transcriptome count scale from 0 to 100. The human transcriptome may be observed only in an "Exp" sample. In addition, the fourth column represents a threshold of 'is_human', defined as a mean plus ten times a standard deviation of %human in a 'Con' sample, and demonstrated a significant number of spots, including the human transcriptome detectable only in the 'Exp'. Therefore, it was confirmed that statistically significant detection of the human transcriptome was made on a Visium platform through hMSC cell injection.

FIG. 11 is a diagram illustrating the result (left) of clustering three samples, that is, the 'Nor', 'Con', and 'Exp', respectively, based on a mouse transcriptome and the result (right) of clustering three samples, that is, the 'Nor', 'Con', and 'Exp', respectively, based on a human transcriptome according to one implementation example of the present disclosure.

FIG. 12 illustrates the results of integrating and clustering spots in three samples, 'Nor', 'Con', and 'Exp', according to one implementation example of the present disclosure.

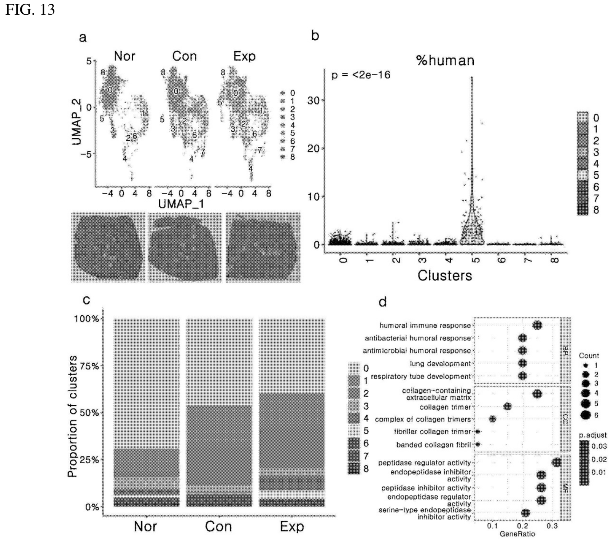

FIG. 13 is a diagram illustrating the results of analyzing clustering for three samples, 'Nor', 'Con', and 'Exp', according to one implementation example of the present disclosure. FIG. 13A is a diagram illustrating the results of DimPlot (top) and SpatialDimplot (bottom) according to cluster number for each sample. FIG. 13B is a diagram illustrating VlnPlot, which shows a proportion (%human) of human transcriptomes among the total transcriptomes according to cluster number (horizontal axis). Interestingly, cluster 5 showed a significantly larger number of human transcriptomes than other clusters. FIG. 13C illustrates the proportion of specific cluster numbers for each sample, and Cluster 5 was barely present in the 'Nor' and 'Con' samples. FIG. 13D is a GO plot for the top 20 spatially enriched genes upregulated according to biological process (BP), cellular component (CC), and molecular function (MF) in cluster 5, in which the gene includes Lcn2 (highest log FC), Msln, Chil3, C3, Upk3b, Spp1, Col4a1, Serpina3n, Wfdc21, Col3a1, Wfdc17, Gm13889, Chil1, Mgp, Sftpd, Slpi, Ctsc, Fmo2, Scd1, and Napsa (in that order).

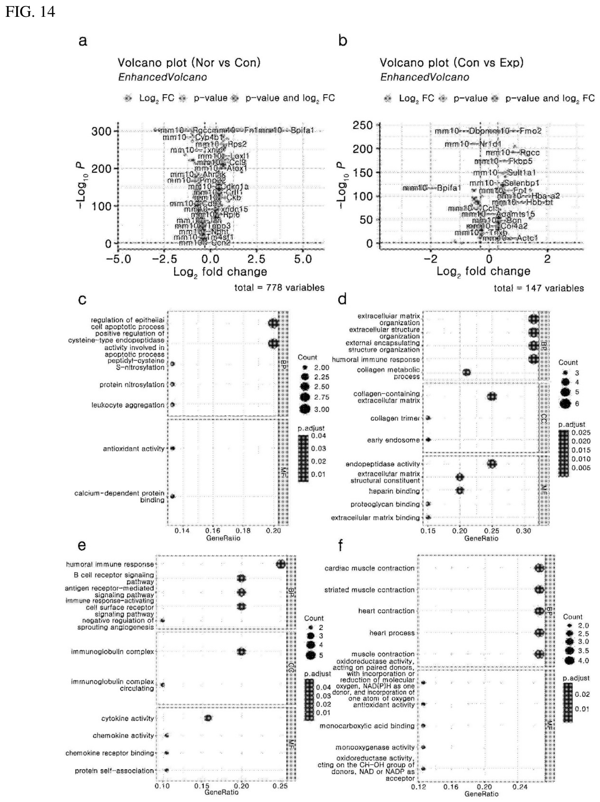

FIG. 14 illustrates transcriptome comparison results of the 'Con' sample and other samples, 'Nor' and 'Exp', according to one implementation example of the present disclosure. FIG. 14a is an EnhancedVolcano plot comparing 'Nor' and 'Con', in which 'Log$_2$ fold change' > 0 indicates genes enriched in the 'Con' sample. FIG. 14B is an EnhancedVolcano plot comparing 'Con' and 'Exp', in which 'Log$_2$ fold change' > 0 indicates genes enriched in the 'Exp' sample. Here, genes tagged with 'mm10' and 'GRCh38' are mouse- and human-derived genes, respectively. FIG. 14C is a GO plot for the top 20 DEGs (adjusted p-value < 0.05, log FC order) enriched in 'Nor' compared to 'Con'. FIG. 14D is a GO plot for the top 20 DEGs (adjusted p-value < 0.05, log FC order) enriched in 'Con' compared to 'Nor'. FIG. 14E is a GO plot for the top 20 DEGs (adjusted p-value < 0.05, log FC order) enriched in 'Con' compared to 'Exp'. FIG. 14F is a GO plot for the top 20 DEGs (adjusted p-value < 0.05, log FC order) enriched in ' Exp' compared to ' Con'.

FIG. 15 is a diagram illustrating the top 6 DEGs (adjusted p-value < 0.05, log FC order) enriched in 'Nor' compared to 'Con'.

FIG. 16 is a diagram illustrating the top 6 DEGs (adjusted p-value < 0.05, log FC order) enriched in 'Con' compared to 'Nor'.

FIG. 17 is a diagram illustrating the top 6 DEGs (adjusted p-value < 0.05, log FC order) enriched in 'Con' compared to 'Exp'.

FIG. 18 is a diagram illustrating the top 6 DEGs (adjusted p-value < 0.05, log FC order) enriched in 'Exp' compared to 'Nor'.

FIG. 19 is a diagram illustrating the proportion of genes spatially associated with the human transcriptome, in which FIG. 19A illustrates the top 20 genes spatially associated with %human in Exp, and FIG. 19B illustrates the bottom 20 genes. FIG. 19C illustrates the GO analysis for mouse genes among the top 20 spatially associated genes for % human.

FIG. 20 is a diagram illustrating the CellDART results. FIG. 20A illustrates the spatial mapping results for the proportion of six cell types in the 'Exp' sample, along with the human transcriptome and %human distribution. FIG. 20B is a correlation coefficient matrix comparing the %human and each mouse cell type ratio predicted by CellDART. Spearman correlation coefficients were used, and as a result, endothelial cells and epithelial cells were the most and least correlated cell types, respectively, with %human.

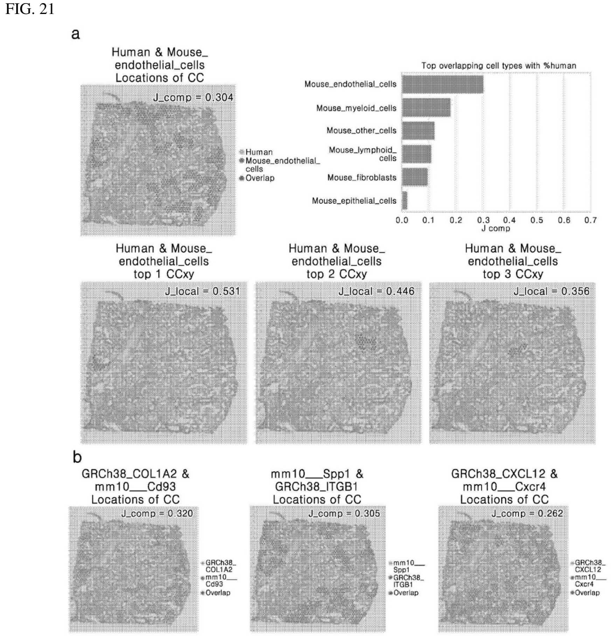

FIG. 21 is a diagram illustrating the STopover results, and specifically, FIG. 21A illustrates the STopover results comparing the spatial distribution of cell types. Here, the CellDART score, calculated by multiplying %human and (100 - %human), was used to represent the spatial distribution between human and mouse cells. J_comp and J_local in the plot represent the global and local Jaccard indices, respectively. In addition, CC also indicates a connected component. FIG. 21B illustrates the topology of the LR pair with the highest Jaccard index for LR co-localization, in which the yellow, blue, and green spots correspond to ligand, receptor, and mixed regions, respectively.

FIG. 22 shows the STopover results derived from a comparison between the distribution of each cell type and % human. Here, the yellow, blue, and green spots correspond to ligand, receptor, and mixed regions, respectively, and the cell types are represented in order of the global Jaccard index (J_comp).

FIGS. 23 and 24 illustrate CellDART results for aDCs, Adipocytes, B cells, Basophils, CD4 T cells, and CD8 T cells among mouse cells in three samples, 'Nor', 'Con', and 'Exp', according to one implementation example of the present disclosure.

FIGS. 25 and 26 are diagrams illustrating the CellDART results for cDC, Chondrocytes, DC, Endothelial cells, Eosinophils, and Erythrocytes among mouse cells in three samples, 'Nor', 'Con', and 'Exp', according to one implementation example of the present disclosure.

FIGS. 27 and 28 are diagrams illustrating the CellDART results for Monocytes, mv. Endothelial cells, Myocytes, Neutrophils, NK cells, and NKT among mouse cells in three samples, 'Nor', 'Con', and 'Exp', according to one implementation example of the present disclosure.

FIGS. 29 and 30 are diagrams illustrating the CellDART results for Osteoblasts, pDCs, Pericytes, Sebocytes, Skeletal muscle, and Smooth muscle among mouse cells in three samples, 'Nor', 'Con', and 'Exp', according to one implementation example of the present invention.

FIGS. 31 and 32 are diagrams illustrating the CellDART results for Tgd cells, Th1 cells, Th2 cells, Tregs, Immune Score, and StromaScore among mouse cells from three samples, 'Nor', 'Con', and 'Exp', according to one implementation example of the present disclosure.

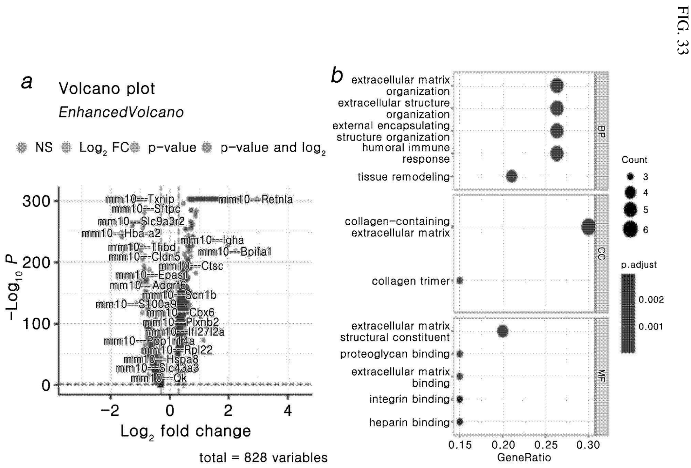

FIG. 33A is a diagram illustrating an EnhancedVolcano plot comparing 'Con' and 'Exp', in which 'Log$_2$ fold change' > 0 indicates genes enriched in the 'Exp' sample. FIG. 33B is a GO plot for the top 20 DEGs (adjusted p-value <0.05, log FC order) enriched in 'Exp' compared to 'Con'.

FIG. 34 is a diagram showing an exploration apparatus 100 according to one aspect of the present disclosure.

FIG. 35 is a block diagram illustrating components constituting the exploration apparatus 100 of FIG. 34.

FIG. 36 is a block diagram illustrating components constituting a molecular marker exploration unit 130 of the exploration apparatus 100 of FIG. 34.

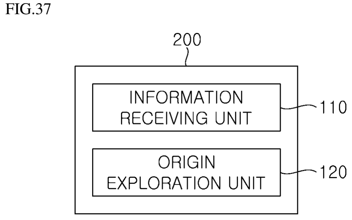

FIG. 37 is a diagram showing a classification apparatus 200 according to one aspect of the present disclosure.

[Mode for Invention]

A first aspect of the present disclosure provides

a method for exploring distribution, efficacy, action, or physiological activity of a genome-containing material within a tissue of interest, including:

obtaining spatially resolved transcriptome information of the tissue of interest by providing a genome-containing material of part or all of a genome not present in the tissue of interest to the tissue of interest that has or is induced by a disease or illness (S10); and

classifying tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs (S20); and

exploring the distribution, efficacy, action, or physiological activity of the genome-containing material within the tissue of interest from the classified information (S30).

In the step (S10) of obtaining spatially resolved transcriptome information of a tissue of interest, part or all of the genome-containing material not present in the tissue of interest may be used without limitation. For example, the genome-containing material may be a cell derived from one or more species selected from the group consisting of umbilical cord, bone marrow, adipose tissue, blood, cord blood, liver, skin, gastrointestinal tract, placenta, and uterus, specifically, a stem cells. This may be homologous or heterologous to the origin species of the tissue of interest, and in a specific example, heterologous. When the genome-containing material is homologous to the origin species of the tissue of interest, the genome-containing material may be a material having a sequence that differs partially or entirely from the native genome by genetically modifying one or more base sequences of the native genome of the tissue of interest through genetic manipulation, modification, deletion, insertion, or the like.

The genome-containing material may be derived from a mammal. The mammal may be a human, monkey, chimpanzee, dog, cat, cow, goat, pig, mouse, or rat, and more specifically, may be a genome-containing material derived from the human, rat, or mouse. The genome-containing material may be a microorganism, cell, stem cell, extracellular vesicle, vector, vehicle, virus, etc. containing a genome. The genome includes a coding sequence encoding a protein and a non-coding sequence, and may be a target for exploring therapeutic effects or action mechanisms, or may be a candidate material for screening therapeutic agents.

The tissue of interest may be, but is not limited to, skin, intestines such as small or large intestine, heart, lung, kidney, liver, spleen, muscle, or tumor tissue. The tissue of interest is a tissue of interest that has the disease or illness of interest or is induced by the disease or illness. The disease or illness of interest is not particularly limited and may include various cancers, brain diseases, neurological diseases, liver diseases, intestinal diseases, fibrosis, immune diseases, viral diseases, kidney diseases, inflammatory diseases, metabolic diseases, skin diseases, diabetes, infectious diseases, cardiovascular diseases, neurodegenerative diseases, and the like, and more specifically, may include cancer, brain diseases, diabetes, inflammatory diseases, viral diseases, infectious diseases, pulmonary fibrosis, and the like.

Providing the genome-containing material to the tissue of interest may include directly providing the genome-containing material to the tissue of interest or systemically administering (e.g., intravenously injecting) the genome-containing material to a subject and then distributing the genome-containing material to the tissue of interest.

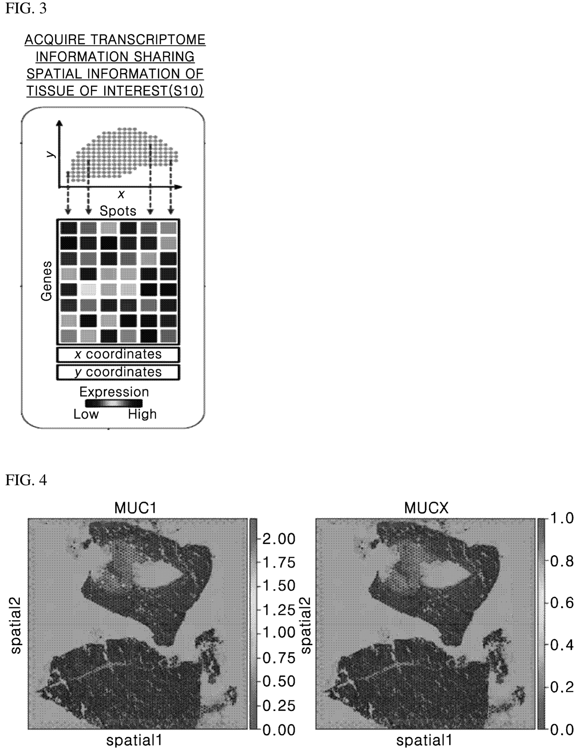

Providing the genome-containing material to the tissue of interest causes the genome to be expressed in the tissue of interest. Accordingly, the tissue of interest includes both a genome-derived transcriptome and a tissue of interest-derived transcriptome, thereby obtaining transcriptome information that shares spatial information of the tissue of interest. The spatially resolved transcriptome information is a technology that provides expression information for hundreds to tens of thousands of genes at a time, and obtains full-length or partial gene expression along with spatial information (see FIG. 3). The spatially resolved transcriptome information may be analyzed by performing a step of fixing a frozen section of the tissue of interest in a mold, and performing steps such as permeabilization, cDNA synthesis, and RNA sequencing. The section of the tissue of interest for obtaining the spatially resolved transcriptome information may optionally be a tissue section that is 'contiguous' with a section for obtaining a stained image of the tissue of interest. The spatially resolved transcriptome information may be the number and type of transcriptome RNA reads within a spot having spatial coordinates within the tissue of interest. Examples of RNA types include information such as whether gene expression, exons, or introns are examined, whether event-based alternative splicing or isoform-based alternative splicing is included, whether mutation burden is examined, and whether noncoding RNA or nontranslated RNA is included.

Thereafter, in the step (S20) of classifying tissue-of-interest-derived transcriptome information and genome-derived transcriptome information among the spatially resolved transcriptome information, an integrated reference transcriptome library may be utilized, including first organism reference transcriptome information to which the tissue of interest belongs and second organism reference transcriptome information to which the genome or genome-containing material belongs. The origin information from the integrated reference transcriptome library may be classified, for example, using SpaceRanger.

The integrated reference transcriptome library may be obtained by integrating the first organism reference transcriptome information to which the tissue of interest belongs with the second organism reference transcriptome information to which the genome-containing material belongs, or vice versa, or may be obtained from the transcriptome information obtained by integrating the first and second organisms. The first and second organisms may be the same or different, and in a specific example, may be different. Using the integrated reference transcriptome library has the advantage of very high matching accuracy, compared to classifying the origin by applying the first organism reference transcriptome information and the second organism reference transcriptome information separately to the spatially resolved transcriptome information. In other words, classifying the origin using the integrated reference transcriptome library allows for identifying the origin of spatially resolved transcriptome information with greater accuracy compared to mapping each reference separately. For example, when the tissue of interest is a tissue of a mouse and the genome-containing material is a human-derived cell, the first organism reference transcriptome information is mm10, the second organism reference transcriptome information is human reference information GRCh38, and the integrated reference transcriptome library refers to information in which mm10 and GRCh38 are combined. Alternatively, the integrated reference transcriptome library may be, for example, a merged single-cell RNA sequencing library in the form of a block diagonal matrix that includes mouse reference transcriptome information (GSE124872) and human reference transcriptome information (GSE147287).

An example of a method for generating the above-mentioned merged single-cell RNA sequencing library is using

$$\begin{pmatrix} A & 0 \\ 0 & B \end{pmatrix},$$

where a human scRNA-seq reference and a mouse scRNA-seq reference may be combined as described above. Here, A represents the human scRNA-seq matrix, with a row name representing a gene name and a column name representing a barcode name, and B represents the mouse scRNA-seq matrix, with a row name representing a gene name and a column names representing a barcode names. Applying a cell type matching algorithm in the tissue of interest to the integrated reference transcriptome library may identify a cell type associated with the distribution of the genome-containing material.

Reference transcriptome information for each organism may be obtained from a publicly available reference transcriptome information provider site or from publicly available resources, based on the origin information of the tissue of interest or the genome-containing material. The integrated reference transcriptome library may also be obtained using the publicly available methods. The above examples include the following: ncbi (e.g., E. coli), etc. The E. coli reference may be found at https://www.ncbi.nlm.nih.gov/assembly/GCF_000005845.2/?shouldredirect=false , and references for other species may also be found on this website.

By matching the obtained spatially resolved transcriptome information with the integrated reference transcriptome library, the spatially resolved transcriptome information may be categorized into the tissue-of-interest-derived transcriptome information and the genome-derived transcriptome information. In other words, by matching the spatially resolved transcriptome information with the integrated reference transcriptome library, the spatially resolved transcriptome information tagged with origin information may be obtained. In this way, it is possible to simply and accurately identify the distribution of genome-containing material within the tissue of interest using the origin information tagged to the genome-derived transcriptome information, without the need for complex procedures such as PCR. For example, in a tissue including cells derived from different origins, or in a tissue of the same origin but comprising gene-modified genome-containing material, it is possible to determine where or to what extent the gene-modified genome-containing material or the cells derived from different origins infiltrate or distribute, which may be utilized as tissue biodistribution data for administered cells for cell therapy.

For tissues containing a gene-modified genome-containing material from the same origin, a method of constructing an integrated reference is as follows.

The basic method for constructing a custom reference is well described in the official documentation provided by 10x Genomics, titled "Build a Custom Reference (cellranger mkref)." For example, a method for constructing a custom reference by inserting the GFP gene (derived from jellyfish) into the human genome reference is as follows:

First, a GFP.fa file containing the following contents is prepared, and may be input using Linux commands like cat and echo, or manually.

>GFP

```
        TACACACGAATAAAAGATAACAAAGATGAGTAAAGGAGAAGAACTTTTCACT
GGAGTTGTCCCAATTCTT
        GTTGAATTAGATGGCGATGTTAATGGGCAAAAATTCTCTGTCAGTGGAGAGGG
TGAAGGTGATGCAACAT
        ACGGAAAACTTACCCTTAAATTTATTTGCACTACTGGGAAGCTACCTGTTCCAT
GGCCAACACTTGTCAC
        TACTTTCTCTTATGGTGTTCAATGCTTTTCAAGATACCCAGATCATATGAAACAG
CATGACTTTTTCAAG
        AGTGCCATGCCCGAAGGTTATGTACAGGAAAGAACTATATTTTACAAAGATGA
CGGGAACTACAAGACAC
        GTGCTGAAGTCAAGTTTGAAGGTGATACCCTTGTTAATAGAATCGAGTTAAAA
GGTATTGATTTTAAAGA
        AGATGGAAACATTCTTGGACACAAAATGGAATACAACTATAACTCACATAATGT
ATACATCATGGCAGAC
        AAACCAAAGAATGGAATCAAAGTTAACTTCAAAATTAGACACAACATTAAAG
ATGGAAGCGTTCAATTAG
        CAGACCATTATCAACAAAATACTCCAATTGGCGATGGCCCTGTCCTTTTACCAG
ACAACCATTACCTGTC
        CACACAATCTGCCCTTTCCAAAGATCCCAACGAAAAGAGAGATCACATGATCC
TTCTTGAGTTTGTAACA
        GCTGCTGGGATTACACATGGCATGGATGAACTATACAAATAAATGTCCAGACTT
CCAATTGACACTAAAG
        TGTCCGAACAATTACTAAATTCTCAGGGTTCCTGGTTAAATTCAGGCTGAGACT
TTATTTATATATTTAT
        AGATTCATTAAAATTTTATGAATAATTTATTGATGTTATTAATAGGGGCTATTTTC
TTATTAAATAGGCT
        ACTGGAGTGTAT
```

Second, a GFP.gtf file containing the following contents is prepared. In this case, the Linux commands like cat and echo may be used, or input manually:

GFP unknown exon 1 922 . + . gene_id "GFP"; transcript_id "GFP"; gene_name "GFP"; gene_biotype "protein_coding";

Third, the contents of the GFP.fa file are inserted into the human_genome.fa file, which stores the base sequence portion of the human reference file, using the following Linux command:

cat GFP.fa >> human_genome.fa

Fourth, the contents of the GFP.gtf file are inserted into the human_genome.gtf file, which stores the gene annotation portion of the human reference file, using the following Linux command:

cat GFP.gtf >> human_genome.gtf

Fifth, a human + GFP custom reference may be generated using the SpaceRanger mkref command:

spaceranger mkref --genome=human_plus_GFP --fasta=human_genome.fa-genes=human_genome.gtf

Subsequently, in exploring step (S30), the classified information (information tagged with the origin information) may be used to explore the distribution, efficacy, action, or physiological activity of the genome-containing material within the tissue of interest.

The exploration may include, for example, extracting molecular markers spatially associated with the ratio of the genome-derived transcriptome counts to the total spatial transcriptome count of the tissue of interest from the spatially resolved transcriptome information tagged with the origin information. The molecular markers may be a single molecule derived from DNA, RNA, metabolites, proteins, protein fragments, etc., or molecular information based on patterns thereof. Extracting the spatially associated molecular markers may utilize differentially expressed genes (DEGs), correlation analysis based on correlation coefficient calculation, an image similarity evaluation algorithm, etc. The correlation coefficient calculation may utilize, for example, a Pearson correlation coefficient, a Spearman correlation coefficient, or a Kendall correlation coefficient. The distribution, efficacy, action, or physiological activity of the genome-containing material within the tissue of interest may be explored from the molecular markers.

For the exploration, the spatially resolved transcriptome image of the classified information or the entire spatially resolved transcriptome image may be segmented into one or more clusters. The one or more clusters may be classified by the intensity of the spatially resolved transcriptome image of the tissue of interest, or may be classified by an algorithm that segments the spatially resolved transcriptome image of the tissue of interest into a plurality of patches and classifies the patches by the similarity of the image features for each patch. For example, the spatially resolved transcriptome image of the tissue of interest may be segmented into patches of $394 \times 384$, each patch having a size of 5x5, 512 features may be extracted from each patch, and the tissue may be classified into one or more clusters based on the features. The tissue of interest image may be classified into cluster 1, cluster 2, cluster 3, etc., and the total number of clusters may be classified into 1 or more, specifically, 2, 3, 4, 5, 6, 7, 8, 9, 10, 11, 12, 13, 14, 15, 16, 17, 18, 19, or 20 clusters, but is not limited thereto. The exploration may include a comparison between one or more clusters, such as selecting a cluster with a significantly higher number of transcriptomes derived from genome-containing material compared to other clusters and comparing the selected cluster's transcriptomes with those of other clusters. For example, when the number of transcriptomes derived from genome-containing material in cluster 4 among clusters 1 to 6 was significantly high and genes correlated with cluster 4 were found, if genes related to hypoxia, glucose metabolism, and cell death mechanisms were dominant, it can be inferred that Cluster 4, due to genome-containing material, is in a hypoxic state, leading to cell death.

For the exploration, a cell type analysis algorithm or a gene ontology (GO) analysis may be performed on the spatially resolved transcriptome information of the classified information or the entire spatially resolved transcriptome information. The cell type analysis algorithm may use Fisher's exact test, maximum likelihood estimation, domain adaptive classification, logistic regression analysis, or negative binomial regression analysis algorithm, and more specifically, the known cell type analysis algorithms, such as the CellDART algorithm, spSeudoMap algorithm, xCell algorithm, RCTD algorithm, Seurat algorithm, celltypist algorithm, cell2location algorithm, Scanorama algorithm, SPOTlight algorithm, DSTG algorithm, CellTrek algorithm, sc-type algorithm, TIMER algorithm, CIBERSORT algorithm, MCP-counter algorithm, Quantiseq algorithm, EPIC algorithm, or scanpy's injest algorithm, MIA algorithm, or STopover algorithm, may be used.

For the exploration, one or more of the following methods may be combined: extraction of spatially correlated molecular markers with the above-described genome-derived transcriptome ratio, one or more clustering methods, image feature analysis, cell type analysis algorithms, and gene ontology analysis. In one example, after performing each of the analysis methods, the results may be synthesized to explore the distribution, efficacy, action, or physiological activity of the genome-containing material within the tissue of interest.

According to another second aspect, the first aspect may further include obtaining comparative transcriptome information of a second tissue of interest that has or is caused by the same disease or illness as the tissue of interest without providing the genome-containing material, and using the comparative transcriptome information in the exploration. The second aspect differs from the first aspect only in the additional use of comparative transcriptome information without the provision of genome-containing material, and the other processes may be performed in the same manner as the above-described aspect. That is, steps S20 and S30 in the above-described aspect may be performed identically for comparative transcriptome information of the second tissue of interest.

Specifically, the second tissue-of-interest-derived transcriptome information and the genome-derived transcriptome information among the comparative transcriptome information are classified (S20') using the integrated reference transcriptome library that includes the first organism reference transcriptome information to which the second tissue of interest belongs and the second organism reference transcriptome information to which the genome or genome-containing material belongs, and the classified information may then be used in step S30 to explore the distribution, efficacy, action, or physiological activity of the genome-containing material within the tissue of interest.

Since the comparative transcriptome information does not provide genome-containing material, step S20' may or may not be omitted. However, if performed, the genome-derived transcriptome information is expected to be absent. The second tissue of interest may have the same origin as the tissue of interest in the above-described aspect.

The specific exploration methods of S30 described above, for example, one or more of the clustering methods, cell type analysis algorithms, and gene ontology analysis methods, may be equally performed on the comparative transcriptome information. In one example, after performing each of the analysis methods, the results may be used to explore the distribution, efficacy, action, or physiological activity of the genome-containing material within the tissue of interest. For example, in step S30, compared to the comparative transcriptome information, the top or bottom expressed transcriptome information may be extracted from the spatially resolved transcriptome information (e.g., the spatially resolved transcriptome information to which the origin information is tagged or not tagged) within the tissue of interest of the genome-containing material, or conversely, compared to the spatially resolved transcriptome information(e.g., the spatially resolved transcriptome information to which the origin information is tagged or not tagged), the top or bottom expressed transcriptome information may be extracted from the comparative transcriptome information. For example, in step S30, the cell type analysis results of the comparative transcriptome information and the cell type analysis results of the spatially resolved transcriptome information (e.g., the spatially resolved transcriptome information to which the origin information is tagged or not tagged) in the tissue of interest of the genome-containing material may be compared with each other, and the results may be utilized for exploration in step S30. For example, in step S30, one or more clusters of the comparative transcriptome information and one or more clusters of the spatially resolved transcriptome information (e.g., the spatially resolved transcriptome information to which the origin information is tagged or not tagged) in the tissue of interest of the genome-containing material may be compared with each other, and the results may be utilized for exploration in step S30.

According to another third aspect, the first and/or second aspect may further include obtaining normal transcriptome information of a normal third tissue of interest that does not have or is not caused by the disease or illness and using the obtained normal transcriptome information for the exploration. The third aspect differs from the above-described aspects only in that no genome-containing material is provided and normal transcriptome information of a normal tissue of interest that does not have or is not caused by the disease or illness is additionally used, and the other processes may be performed in the same manner as in the above-described aspects. That is, steps S20 (or S20') and S30 in the above-described aspects may be performed in the same manner for the normal transcriptome information of the third tissue of interest.

Specifically, the third tissue-of-interest-derived transcriptome information and the genome-derived transcriptome information among the normal transcriptome information is classified (S20") using the integrated reference transcriptome library that includes the first organism reference transcriptome information to which the third tissue of interest belongs and the second organism reference transcriptome information to which the genome or genome-containing material belongs, and the classified information may then be used in step S30 to explore the distribution, efficacy, action, or physiological activity of the genome-containing material within the tissue of interest.

Since the normal transcriptome information does not provide the genome-containing material, step S20" may or may not be omitted. However, if performed, the genome-derived transcriptome information is expected to be absent. The third tissue of interest may have the same origin as the first or second tissue of interest in the above-described aspect.

The specific exploration methods of S30 described above, for example, one or more of the clustering methods, cell type analysis algorithms, and gene ontology analysis methods, may be equally performed on the normal transcriptome information. In one example, after performing each of the analysis methods, the results may be used to explore the distribution, efficacy, action, or physiological activity of the genome-containing material within the tissue of interest. For example, in step S30, compared to the normal transcriptome information, the top or bottom expressed transcriptome information may be extracted from the spatially resolved transcriptome information (e.g., the spatially resolved transcriptome information to which the origin information is tagged or not tagged) within the tissue of interest of the genome-containing material, the top or bottom expressed transcriptome information may be extracted from the comparative transcriptome information, or the top or bottom expressed transcriptome information may be extracted from the normal transcriptome information by comparing the normal transcriptome information with the spatially resolved transcriptome information (e.g., the spatially resolved transcriptome information to which the origin information is tagged or not tagged) or the comparative transcriptome information. For example, in step S30, the cell type analysis results of the normal transcriptome information may be compared with the cell type analysis results of the spatially resolved transcriptome information (e.g., the spatially resolved transcriptome information to which the origin information is tagged or not tagged) within the genome-containing material's tissue of interest and/or the cell type analysis results of the comparative transcriptome information, and the results may be used for exploration in step S30. For example, in step S30, one or more clusters of the normal transcriptome information may be compared with one or more clusters of the spatially resolved transcriptome information (e.g., the spatially resolved transcriptome information to which the origin information is tagged or not tagged) within the tissue of interest of the genome-containing material and/or one or more clusters of the comparative transcriptome information, and the results may be used for exploration in step S30.

The exploration in step S30 may further include comparing one or more of the spatially resolved transcriptome information, the comparative transcriptome information, and the normal transcriptome information within the tissue of interest to identify the molecular markers derived from the genome-containing material that are enhanced or suppressed.

According to another fourth aspect, any one of the above-described aspects may further include staining any one or more of the tissue of interest, the second tissue of interest, and the third tissue of interest, and obtaining the stained image of each tissue. The staining method may include, but is not limited to, alkaline phosphate assay (ALP assay), Sirius red staining, Alcian blue staining, pH map, H&E staining, Trichrome staining, periodic acid-Schiff (PAS) staining, immunohistochemical staining, etc. The result of the stained tissue image may be used as a complementary or supplementary means for verifying the exploring result according to the present disclosure or extracting useful information when exploring the molecular markers. In one example, the stained tissue image may be used as it is, or an image feature extraction algorithm using artificial intelligence, for example, a spatial gene expression patterns by deep learning of tissue images (SPADE) algorithm may be applied to extract image features, and SPADE gene information related to the extracted features may be obtained.

In another fifth aspect of the present disclosure,

there is provided a method for classifying genome-derived transcriptome information from spatially resolved transcriptome information within a tissue of interest, including:
obtaining spatially resolved transcriptome information of the tissue of interest by providing a genome-containing material of part or all of a genome not present in the tissue of interest to the tissue of interest that has or is induced by a disease or illness; and
classifying genome-derived transcriptome information among spatially resolved transcriptome information using an integrated reference transcriptome library including first organism reference transcriptome information to which the tissue of interest belongs and second organism reference transcriptome information to which the genome or a genome-containing material belongs.

The classification method may have higher classification accuracy compared to classifying genome-derived transcriptome information among spatially resolved transcriptome information using first or second organism reference transcriptome information, without using an integrated reference transcriptome library.

In another sixth aspect of the present disclosure,

there is provided an apparatus 100 for exploring distribution, efficacy, action, or physiological activity of a genome-containing material within a tissue of interest, including: an information receiving unit 110 that obtains spatially resolved transcriptome information of the tissue of interest by providing a genome-containing material of part or all of a genome not present in the tissue of interest to the tissue of interest that has or is induced by a disease or illness, and receives the obtained information;
an origin exploration unit 120 that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs; and
a molecular marker exploration unit 130 that explores a molecular marker related to the distribution, efficacy, action, or physiological activity of the genome-containing material within the tissue of interest from the information of the origin exploration unit 120.

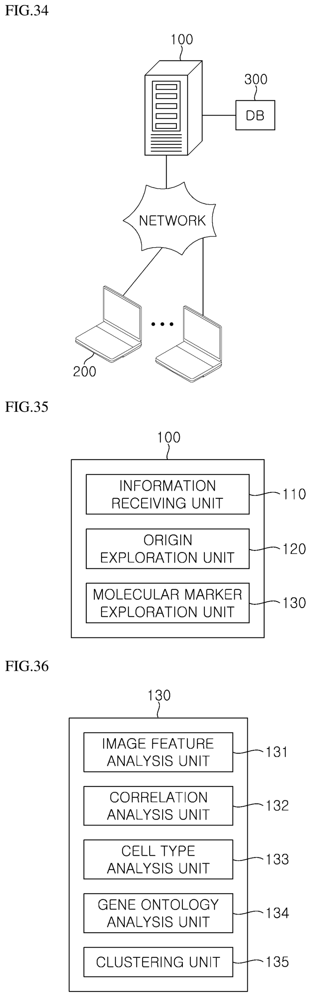

In the seventh aspect, a system including the exploration apparatus 100 of the present disclosure is provided (FIG. 34).

In the sixth and seventh aspects, the 'genome-containing material', the 'tissue of interest', the 'spatially resolved transcriptome information', the 'first organism reference transcriptome information', the 'second organism reference transcriptome information', the 'integrated reference transcriptome library', 'molecular marker', etc., are the same as those described in the above-described aspects, and therefore detailed descriptions thereof are omitted. In the following, description is given for a range that does not overlap.

The information receiving unit 110 receives both the genome-derived transcriptome information and the tissue-of-interest-derived transcriptome information expressed in the tissue of interest upon providing the genome-containing material to the tissue of interest. In other words, the information receiving unit 110 receives transcriptome information that shares the spatial information of the tissue of interest.

The spatially resolved transcriptome information may be analyzed by steps of fixing the frozen section of the tissue of interest in the mold and steps such as permeabilization, cDNA synthesis, and RNA sequencing, and may be the number of transcriptome RNA reads and RNA types within a spot having spatial coordinates within the tissue of interest. Examples of the RNA types include origin information such as whether the RNA is mouse or human RNA and information such as whether gene expression is examined, exons or introns are examined, whether event-based alternative splicing or isoform-based alternative splicing is included, whether mutation burden is examined, and whether noncoding RNA or nontranslated RNA is included. The information receiving unit 110 receives the spatially resolved transcriptome information described above.

The origin exploration unit 120 classifies the spatially resolved transcriptome information into the tissue-of-interest-derived transcriptome information and the genome-derived transcriptome information from the integrated reference transcriptome library containing the first organism reference transcriptome information to which the tissue of interest belongs and the second organism reference transcriptome information to which the genome or genome-containing material belongs. As a result, all the spatially resolved transcriptome information is tagged with its respective origin. That is, the origin exploration unit 120 may obtain the spatially resolved transcriptome information tagged with the origin information by matching the spatially resolved transcriptome information with the integrated reference transcriptome library.

The molecular marker exploration unit 130 may explore the distribution, efficacy, action, or physiological activity of the genome-containing material within the tissue of interest based on the information from the origin exploration unit 120.

The molecular marker exploration unit 130 may include, for example, a correlation analysis unit 132 that extracts the molecular markers spatially correlated with the ratio of the number of genome-derived transcriptomes to the total number of spatial transcriptomes within the tissue of interest from the spatially resolved transcriptome information tagged with the origin information. The correlation analysis unit 132 may be molecular information based on a single molecule derived from DNA, RNA, metabolites, proteins, protein fragments, etc., or patterns thereof. Extracting the spatially associated molecular markers may utilize differentially expressed genes (DEGs), correlation analysis based on correlation coefficient calculation, an image similarity evaluation algorithm, etc. The correlation coefficient calculation may utilize, for example, a Pearson correlation coefficient, a Spearman correlation coefficient, or a Kendall correlation coefficient. The distribution, efficacy, action, or physiological activity of the genome-containing material within the tissue of interest may be explored from the molecular markers.

The molecular marker exploration unit 130 may include a clustering unit 135 that segments the spatially resolved transcriptome image of the classified information or the entire spatially resolved transcriptome image into one or more clusters. The one or more clusters may be classified by the intensity of the spatially resolved transcriptome image of the tissue of interest, or may be classified by an algorithm that segments the spatially resolved transcriptome image of the tissue of interest into a plurality of patches and classifies the patches by the similarity of the image features for each patch. For example, the spatially resolved transcriptome image of the tissue of interest may be segmented into patches of $394 \times 384$, each patch having a size of $5 \times 5$, 512 features may be extracted from each patch, and the tissue may be classified into one or more clusters based on the features. The tissue of interest image may be classified into cluster 1, cluster 2, cluster 3, etc., and the total number of clusters may be classified into 1 or more, specifically, 2, 3, 4, 5, 6, 7, 8, 9, 10, 11, 12, 13, 14, 15, 16, 17, 18, 19, or 20 clusters, but is not limited thereto. The molecular marker exploration unit 130 may include a comparison between one or more clusters, such as selecting a cluster with a significantly higher number of transcriptomes derived from genome-containing material compared to other clusters and comparing the selected cluster's transcriptomes with those of other clusters.

The molecular marker exploration unit 130 may include a cell type analysis unit 133 and/or a gene ontology (GO) analysis unit 134 for the classified spatially resolved transcriptome information or the entire spatially resolved transcriptome information. The cell type analysis unit 133 may use Fisher's exact test, maximum likelihood estimation, domain adaptive classification, logistic regression analysis, or negative binomial regression analysis algorithm, and more specifically, may use the known cell type analysis algorithms such as the CellDART algorithm, the STopover algorithm, the spSeudoMap algorithm, the xCell algorithm, the RCTD algorithm, the Seurat algorithm, the celltypist algorithm, the cell2location algorithm, the Scanorama algorithm, the SPOTlight algorithm, the DSTG algorithm, the CellTrek algorithm, the sc-type algorithm, the TIMER algorithm, the CIBERSORT algorithm, the MCP-counter algorithm, the Quantiseq algorithm, the EPIC algorithm, or scanpy's injest algorithm.

The molecular marker exploration unit 130 may include an image feature analysis unit 131 that extracts the transcriptome information using an image (or imaging) feature extraction algorithm using the artificial intelligence. The image feature analysis unit 131 is configured to extract the transcriptome information using an image (or imaging) feature extraction algorithm using the artificial intelligence, and the image feature extraction algorithm using the artificial intelligence may use a spatial gene expression patterns by deep learning of tissue images (SPADE) algorithm, etc. The SPADE algorithm is known in https://doi.ore/10.1101/2020.06.15.150698, which is incorporated herein by reference in its entirety. The image feature extraction algorithm extracts features from the stained image of the tissue of interest, the transcriptome image of the tissue of interest, or each cluster of the image, and provides the SPADE gene information related to the extracted features. The SPADE algorithm uses a pre-trained VGG16 model to extract, for example, 512 features per patch around each point, perform principal component analysis (PCA) to reduce the dimensionality of the features, and select principal components (PCs) to identify SPADE genes.

The molecular marker exploration unit 130 may include one or more of the image feature analysis unit 131, the correlation analysis unit 132, the cell type analysis unit 133, the gene ontology (GO) analysis unit 134, and the clustering unit 135. In one example, after performing each of the analysis units, the results may be synthesized to explore the distribution, efficacy, action, or physiological activity of the genome-containing material within the tissue of interest.

According to another eighth aspect, the exploration apparatus or system of the sixth or seventh aspects described above further includes a comparative transcriptome information receiving unit 111 that receives comparative transcriptome information of a second tissue of interest that is not provided with the genome-containing material and has or is caused by the same disease or illness as the tissue of interest. The eighth aspect differs from the sixth aspect only in that it additionally uses the comparative transcriptome information receiving unit 111 for which no genome-containing material is provided, and is otherwise the same as the above-described aspects. That is, the comparative transcriptome information receiving unit 111 for the second tissue of interest may apply the origin exploration unit 120 and the molecular marker exploration unit 130 of the sixth aspect and receive the result as the molecular marker exploration unit 130.

Specifically, the origin of the comparative transcriptome information may be explored (120') by the integrated reference transcriptome library including the first organism reference transcriptome information to which the second tissue of interest belongs and the second organism reference transcriptome information to which the genome or genome-containing material belongs, and the classified information may be used in the molecular marker exploration unit 130 of the distribution, efficacy, action, or physiological activity of the genome-containing material within the tissue of interest.

The comparative transcriptome information receiving unit 111 may or may not include the origin exploration unit 120' because it does not provide the genome-containing material. However, in either case, the genome-derived transcriptome information is expected to be absent. The second tissue of interest may have the same origin as the tissue of interest in the above-described aspect.

One or more of the specific exploration methods of the above-described molecular marker exploration unit 130, such as the image feature analysis unit 131, the correlation analysis unit 132, the cell type analysis unit 133, the gene ontology (GO) analysis unit 134, and the clustering unit 135, may be performed identically on the comparative transcriptome information. In one example, after performing each of the analysis methods, the results may be used to explore the distribution, efficacy, action, or physiological activity of the genome-containing material within the tissue of interest. For example, compared to the comparative transcriptome information, the molecular marker exploration unit 130 may extract the top or bottom expressed transcriptome information from the spatially resolved transcriptome information (e.g., the spatially resolved transcriptome information to which the origin information is tagged or not tagged) within the tissue of interest of the genome-containing material, or conversely, extract the top or bottom expressed transcriptome information from the comparative transcriptome information by comparing the comparative transcriptome information with the spatially resolved transcriptome information (e.g., the spatially resolved transcriptome information to which the origin information is tagged or not tagged). For example, the cell type analysis results of the comparative transcriptome information and the cell type analysis results of the spatially resolved transcriptome information (e.g., the spatially resolved transcriptome information to which the origin information is tagged or not tagged) in the tissue of interest of the genome-containing material may be compared with each other, and the results may be utilized for the exploration of the molecular marker exploration unit 130. For example, one or more clusters of the comparative transcriptome information and one or more clusters of the spatially resolved transcriptome information (e.g., the spatially resolved transcriptome information to which the origin information is tagged or not tagged) in the tissue of interest of the genome-containing material may be compared with each other, and the results may be utilized for the exploration of the molecular marker exploration unit 130.

According to another ninth aspect, the sixth and/or eighth aspects may further include a normal transcriptome information receiving unit 112 that obtains normal transcriptome information of a normal third tissue of interest that does not have or is not caused by the disease or illness and does not provide the genome-containing material, and receive the obtained information. The ninth aspect differs from the sixth and eighth aspects only in that it additionally uses the normal transcriptome information receiving unit 112, and is otherwise identical to the above-described aspect. That is, the normal transcriptome information receiving unit 112 for the third tissue of interest may apply the origin exploration unit 120 and the molecular marker exploration unit 130 of the sixth aspect and receive the result as the molecular marker exploration unit 130.

Specifically, the origin of the normal transcriptome information may be explored (120") by the integrated reference transcriptome library including the first organism reference transcriptome information to which the third tissue of interest belongs and the second organism reference transcriptome information to which the genome or genome-containing material belongs, and the classified information may be used in the molecular marker exploration unit 130 of the distribution, efficacy, action, or physiological activity of the genome-containing material within the tissue of interest.

The normal transcriptome information receiving unit 112 may or may not include the origin exploration unit 120"

because it does not provide the genome-containing material. However, in either case, the genome-derived transcriptome information is expected to be absent. The third tissue of interest may have the same origin as the tissue of interest in the above-described aspect.

One or more of the specific exploration methods of the above-described molecular marker exploration unit 130, such as the image feature analysis unit 131, the correlation analysis unit 132, the cell type analysis unit 133, the gene ontology (GO) analysis unit 134, and the clustering unit 135, may be performed identically on the normal transcriptome information. In one example, after performing each of the analysis methods, the results may be used to explore the distribution, efficacy, action, or physiological activity of the genome-containing material within the tissue of interest. For example, compared to the normal transcriptome information, the molecular marker exploration unit 130 may extract the top or bottom expressed transcriptome information from the spatially resolved transcriptome information (e.g., the spatially resolved transcriptome information to which the origin information is tagged or not tagged) within the tissue of interest of the genome-containing material, extract the top or bottom expressed transcriptome information from the comparative transcriptome information, extract the top or bottom expressed transcriptome information from the normal transcriptome information by comparing the normal transcriptome information with the spatially resolved transcriptome information (e.g., the spatially resolved transcriptome information to which the origin information is tagged or not tagged) or the comparative transcriptome information, etc. For example, the molecular marker exploration unit 130 may compare the cell type analysis results of the normal transcriptome information with the cell type analysis results of the spatially resolved transcriptome information (e.g., the spatially resolved transcriptome information to which the origin information is tagged or not tagged) within the genome-containing material's tissue of interest and/or the cell type analysis results of the comparative transcriptome information, and examine the changes in cell types and use the results for exploration. For example, the molecular marker exploration unit 130 may compare one or more clusters of the normal transcriptome information with one or more clusters of the spatially resolved transcriptome information (e.g., the spatially resolved transcriptome information to which the origin information is tagged or not tagged) within the tissue of interest of the genome-containing material and/or one or more clusters of the comparative transcriptome information, and use the results for exploration.

The molecular marker exploration unit 130 may further include exploring the molecular markers derived from the genome-containing material that are enhanced or suppressed by comparing one or more of the tissue of interest of the spatially resolved transcriptome information, the comparative transcriptome information, and the normal transcriptome information.

In another tenth aspect,

there is provided an apparatus 200 for classifying genome-derived transcriptome information from spatially resolved transcriptome information, including: the information receiving unit 110 that obtains the spatially resolved transcriptome information of the tissue of interest by providing the genome-containing material of part or all of a genome not present in the tissue of interest to the tissue of interest that has or is induced by the disease or illness, and receives the obtained information; and

the origin exploration unit 120 that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs.

The apparatus 200 has higher classification accuracy compared to classifying the genome-derived transcriptome information among the spatially resolved transcriptome information using the first or second organism reference transcriptome information, without using the integrated reference transcriptome library.

Furthermore, the exploration apparatus 100 or the classification apparatus 200 may further include a stained tissue image receiving unit 150 that stains at least one of the tissue of interest, the second tissue of interest, and the third tissue of interest and receives the stained images of each tissue.

The staining method may include, but is not limited to, alkaline phosphate assay (ALP assay), Sirius red staining, Alcian blue staining, pH map, H&E staining, Trichrome staining, periodic acid-Schiff (PAS) staining, immunohistochemical staining, etc. The result of the stained tissue image may be used as a complementary or supplementary means for verifying the search result according to the present disclosure or extracting useful information when exploring the molecular markers. In one example, the stained tissue image may be used as it is, or an image feature extraction algorithm using artificial intelligence, for example, a spatial gene expression patterns by deep learning of tissue images (SPADE) algorithm may be applied to extract image features, and SPADE gene information related to the extracted features may be obtained.

The present disclosure will be described in more detail below through examples and analysis examples. However, the following examples and analysis examples are intended solely to illustrate the present disclosure and are not intended to limit the scope of the present disclosure.

Example

Method

Animal Experiments and Tissue Procurement

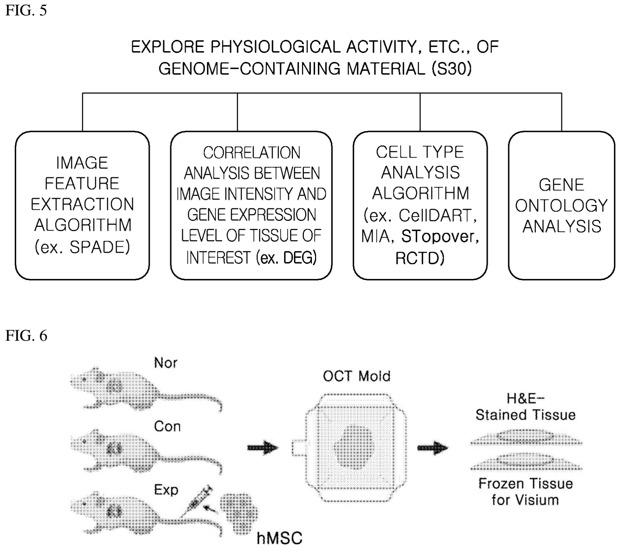

This animal experiment was approved by Seoul National University Bundang Hospital under IACUC approval code BA-2211-355-002. Normal bone marrow-derived hMSCs were prepared from Lonza™. Three groups of C57BL/6 male mice (9 weeks old) were prepared: A normal mouse 'Nor', a mouse 'Con' with pulmonary fibrosis as a control group, and a mouse with pulmonary fibrosis and an experimental group 'Exp' in which hMSCs were injected via a tail vein. In both the control group 'Con' and experimental group 'Exp', pulmonary fibrosis was induced by bleomycin administration for 3 weeks prior to sacrifice. Then, optimal cutting temperature (OCT) blocks (Scigen 4586, USA) were prepared according to the Visium Spatial Protocols - Tissue Preparation Guide (document CG000240). OCT blocks for the 'Exp' group were prepared 6 hours after human stem cell injection. Thereafter, two tissue slices were prepared for each OCT mold in the group, thereby obtaining H&E-stained tissue slices and fresh-frozen tissue slices for the Visium ST library (FIG. 6).

Acquisition of ST Library

Tissue sections were fixed, stained, and permeabilized using the Visium Spatial Protocols - Spatial Gene Expression Imaging Guide (document CG000241), along with the tissue optimization (TO) step. mRNA present in the tissues was captured via poly-A tails, and subsequent cDNA was barcoded and amplified via polymerase chain reaction (PCR) to obtain sufficient cDNA for library reconstruction. Quantitative PCR (qPCR) and the Agilent Technologies 4200 TapeStation were used to measure and evaluate library quality. Finally, the libraries were sequenced using the Illumina HiSeq platform according to the instructions provided in the user guide.

Additionally, SpaceRanger (ver. 2.0.1) mkref was used to merge the representative human reference, GRCh38, and the representative mouse reference, mm10, to obtain the integrated reference transcriptome library. SpaceRanger counts were run on each of the Nor, Con, and Exp samples to generate individual processed ST libraries, which were then mapped to the integrated reference transcriptome library.

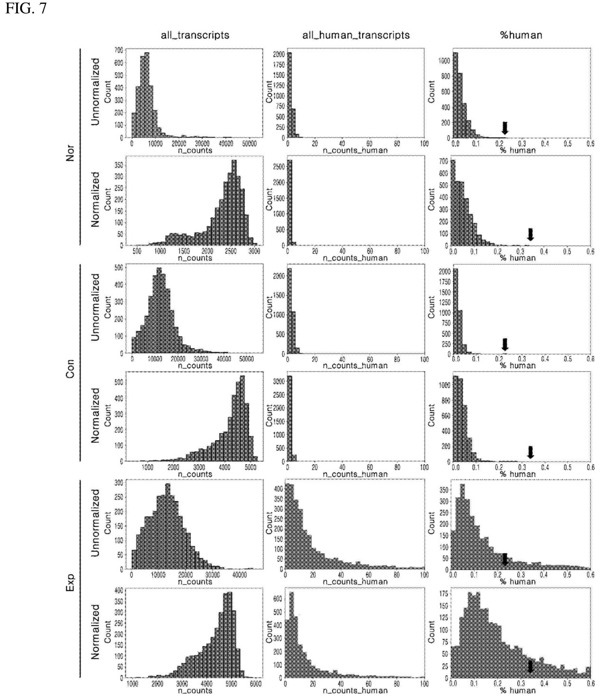

At a given sequencing depth, the raw number of genes at a single spot is inversely proportional to the total RNA production for the corresponding spot. To address this, gene counts were adjusted using various methods, such as trimmed mean of M-values (TMM) and fractile normalization [11]. In addition, the gene counts are typically log-transformed, as large gene counts may overestimate their activity. This process, known as the normalization, may be performed in Python using the scanpy.pp.normalize_total and scanpy.pp.log1p functions. After normalizing the gene counts, unreliably high count values in some RNA transcriptomes were corrected (FIG. 7, first column). %human, the percentage of the human transcriptome relative to the total transcriptome at a single spot, was examined by sample and normalization. As a result, %human with normalization was higher in the 'Exp' sample compared to the unnormalized sample (FIG. 7, bottom right), but had only a minimal effect in the 'Nor' and 'Con' samples. The results demonstrate that the normalization increases the statistical power for detecting human transcriptomes.

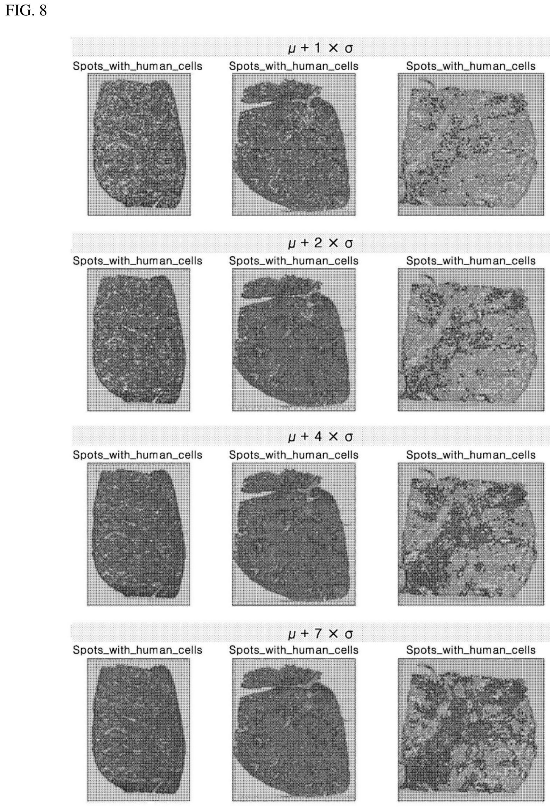

Therefore, in the subsequent analysis, only the human transcriptome and %human were considered after normalization. To address falsely detected human transcriptomes, various thresholds for %human were tested (FIG. 8). As a result, the threshold was defined as the mean plus 10 times the standard deviation of %human in the 'Con' sample for two reasons: First, this threshold is just above the cutoff for %human, which no spots exceed in the 'Nor' and 'Exp' samples (FIG. 7, third column). Second, when applying this threshold, spots classified as human cells in the 'Exp' sample exhibited a histological pattern suitable for comparison in the subsequent cell type matching analysis.

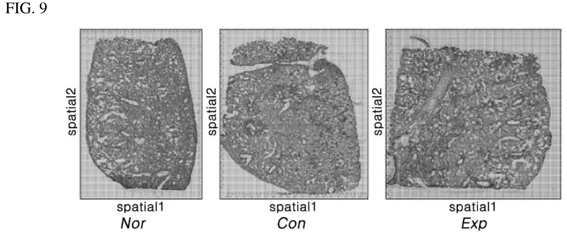

H&E-stained tissue slice images are illustrated in FIG. 9. Con and Exp samples exhibit pulmonary fibrosis, while Nor samples exhibit no pulmonary fibrosis.

The number of transcriptomes per spot (all_transcripts), the number of mouse transcriptomes per spot (all_mouse_transcripts), and the number of human transcriptomes per spot (all_human_transcripts) in the three samples of 'Nor', 'Con', and 'Exp' are illustrated in FIG. 10. That is, FIG. 10 is an image showing the number of all transcriptomes (first column), the number of mouse transcriptomes (second column), and the number of human transcriptomes (third column). The fourth column represents binarization of each tissue by defining the mean + $10\sigma$ of the number of transcripts per spot as a cutoff. In 'is_human', the yellow spots here are spots that exceeded the cutoff value, and the purple spots represent spots that did not exceed the cutoff value. Here, the human transcriptome showed a significant number of spots, including the human transcriptome, which was detectable only in 'Exp', on a transcriptional scale from 0 to 100. The distribution of %human on ST in the 'Exp' sample may be considered as the distribution of administered hMSCs (column 4 of FIG. 10). Therefore, it was confirmed that statistically significant detection of the human transcriptome was made on a Visium platform through hMSC cell injection.

Clustering analysis confirming hMSCs administered in lung tissue

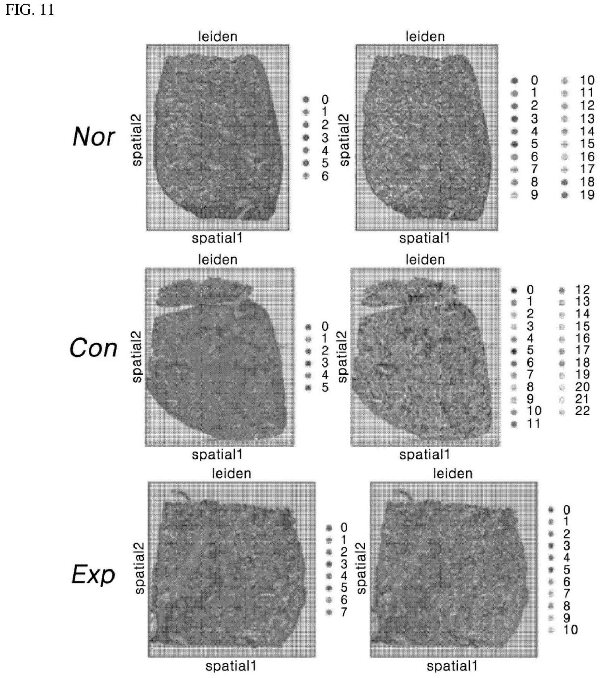

FIG. 11 illustrates the results of clustering based on the mouse transcriptome (left) and the human transcriptome (right) for each of the three samples: 'Nor', 'Con', and 'Exp'.

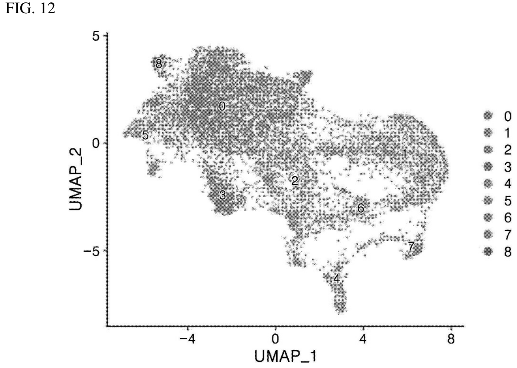

FIG. 12 illustrates the results of clustering analysis by combining spots from the three samples: 'Nor', 'Con', and 'Exp'. Furthermore, the clustering results for each of the three samples 'Nor', 'Con', and 'Exp' are illustrated in FIG. 13A (DimPlot (top) and SpatialDimplot (bottom) of three samples). The VlnPlot showed the proportion of human transcriptomes (%human) relative to the total transcriptome by cluster number (horizontal axis). Interestingly, it was showed that cluster 5 contained significantly more human transcriptomes than the other clusters (FIG. 13B). The proportion of specific cluster numbers for each of the three samples is illustrated in FIG. 13C, in which cluster 5 was rarely present in the 'Nor' and 'Con' samples. DEG and GO analyses were performed for cluster 5, and the results were illustrated in FIG. 13D. The specific DEGs of cluster 5, i.e., the top 20 spatially enriched upregulated genes, were Lcn2 (highest log FC), Msln, Chil3, C3, Upk3b, Spp1, Col4a1, Serpina3n, Wfdc21, Col3a1, Wfdc17, Gm13889, Chil1, Mgp, Sftpd, Slpi, Ctsc, Fmo2, Scd1, and Napsa (in that order). These genes were also associated with immune response, collagen-containing extracellular matrix (ECM), and peptidase activity in GO analysis based on biological process (BP), cellular component (CC), and molecular function (MF). This suggests that hMSC distribution contributes to immune response, collagen-containing extracellular matrix (ECM), and peptidase activity (refer to results in FIG. 13D).

Acquisition of DEGs Related to hMSC Distribution

Data integration was performed using FindIntegrationAnchors and IntegrateData in Seurat (version 4.3.0). Thereafter, the spatial clustering analysis was performed after removing human genes from the integrated Seurat entities. This removal process was considered meaningful to avoid biasing the "Exp" entity, which only contains the true human transcriptome. However, this removal process was not performed in other analyses. The top 20 spatially enriched genes (adjusted p-value < 0.05, log FC order) in a given cluster were obtained by Wilcoxon Rank Sum test using Seurat's FindAllMarkers. Five categories based on biological process (BP), cellular component (CC), and molecular function (MF) were obtained by exploring the Gene Ontology (GO) plot of clusterProfiler::enrichGO (ver. 4.6.2) in R.GO plots.

To perform the comparative analysis between samples, differentially expressed genes (DEGs) were obtained by Wilcoxon Rank Sum test using Seurat's FindAllMakers. Then, the top 20 DEGs (adjusted p-value < 0.05, log FC order) were explored in the GO analysis.

To obtain genes spatially associated with %human, the Spearman correlation coefficient between %human and the scaled expression of each gene was calculated only in the 'Exp' sample. Next, the top 20 mouse genes spatially associated with %human (adjusted p-value < 0.05, Spearman correlation order) were explored in the GO analysis.

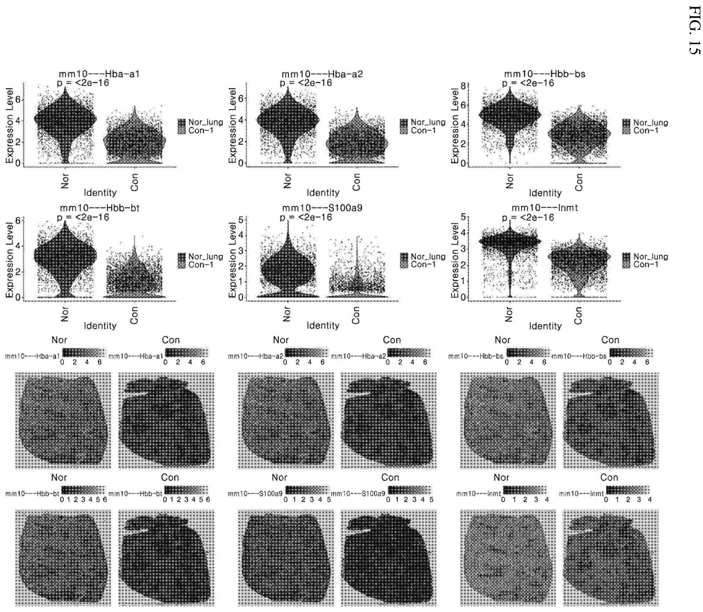

Differentially expressed genes (DEGs) were obtained between 'Con' and 'Exp'. Although all human and mouse genes were included, the top six DEGs were all mouse genes (see FIGS. 15 to 18). To obtain genes spatially associated with hMSC distribution, genes spatially associated with %human in the 'Exp' sample were explored.

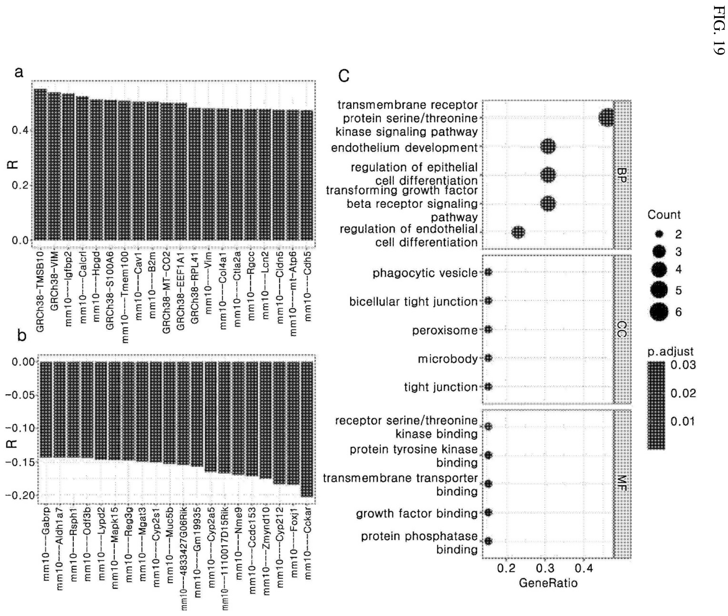

The comparative analysis between samples was performed to obtain the differentially expressed genes (DEGs) using Seurat's FindAllMakers. Thereafter, the top 20 DEGs with positive correlations (adjusted p-value < 0.05, LogFC order) and the bottom 20 DEGs with negative correlations were explored in the GO analysis (see FIGS. 19A and 19B). Six of the top 20 DEGs were human genes. To evaluate the molecular process of pulmonary fibrosis in the 'Exp' sample, positively spatially associated mouse genes among the top 20 DEGs were selected and subjected to the GO analysis.

The biological pathway of the above-described top genes was the "Transmembrane Receptor Protein Serine/-Threonine Kinase Signaling Pathway" (see FIG. 19C). In addition, there were mouse genes that overlapped between the top 20 spatially enriched genes in cluster 5 and the genes spatially associated with %human, namely Lcn2 and Col4al (see FIGS. 13 and 19).

Cell type populations in a lung fibrosis model associated with hMSC treatment were identified using the CellDART.

To identify the cell types spatially associated with the distribution of %human, cell type inference using domain adaptation of single-cell and spatial transcriptome data (CellDART) was performed [12]. A single-cell RNA sequencing (scRNA-seq) reference was generated using the publicly available mouse lung scRNA-seq reference (GSE124872). Spearman correlation coefficients were calculated between the CellDART score and %human for each mouse cell type using only the "Exp" sample.

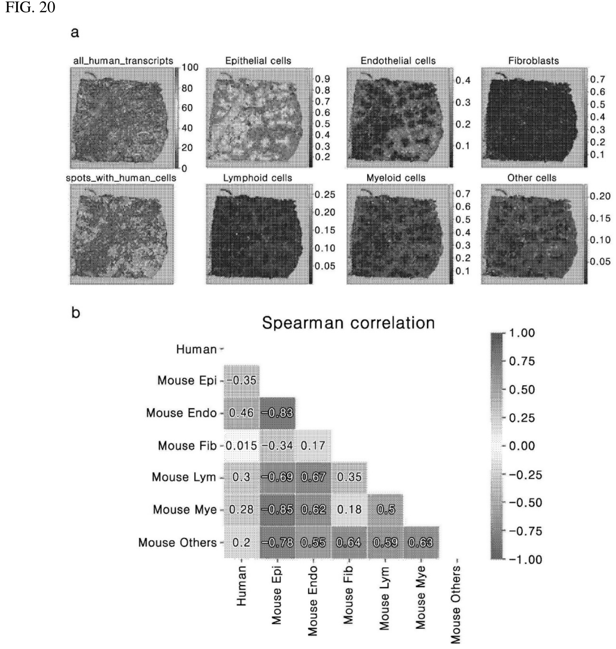

More specifically, after preparing a single-cell RNA sequencing (scRNA-seq) reference of mouse lung (GSE124872), cell-level deconvolution was performed on the 'Exp' sample using CellDART [12] (FIG. 20A). The Spearman correlation between %human and CellDART scores for each cell type in the processed lung tissue was calculated (FIG. 20B). Endothelial cells were most closely correlated with %human, while epithelial cells were the least correlated cell type to %human (FIG. 20B). This is consistent with the idea that intravenously injected cells are physically trapped in the narrow microvasculature of the alveoli during the circulation [13].

We defined the 'is_endothelial' variable to indicate whether the spot in the 'Exp' sample was composed of endothelial cells, using the average threshold of the CellDART score for the endothelial cells plus one standard deviation. Thereafter, regardless of whether they were designated as "is_human", spots labeled as "is_endothelial" (referred to simply as "endothelial spots") were compared to obtain DEGs using the same method as the previous DEG analysis. As a result, only Apoe, Col1a1, and Hnrnpab were significantly enriched in low-%human endothelial spots (adjusted p-value < 0.05). Among them, Apoe and Col1a1 (an activated fibroblast marker) are known to be localized to fibrotic regions around blood vessels [10]. In contrast, mt-Co2, Lcn2, Gm42418, Chil1, Scd1, mt-Nd1, and mt-Nd2, along with 43 human genes, were significantly enriched in high-%human endothelial spots (adjusted p-value < 0.05). Interestingly, Lcn2, which was identified in the previous two analyses, also appeared in this analysis, thereby supporting the overall analysis.

The STopover analysis was used to identify co-localized cell types based on %human and interspecies LR interactions.

The STopover was introduced as a method to measure the spatial co-localization of two continuous variables in ST barcodes [16]. This algorithm calculates the Jaccard index evaluating $|A \cap B|$, and given sets A and B, the Jaccard index is divided by $|A \cup B|$. The index may be calculated globally (J_comp) or locally (J_local), and the latter depends on connected components (CC). STopover parameters were set to default. In addition, the Stopover was used to explore the spatial associations of cell types. Here, to represent the spatial distribution of human and mouse cells, respectively, the CellDART score obtained by multiplying %human and (100 - %human) was used.

In addition, the ligand-receptor interaction between different species was investigated. The existing ligand-receptor database, including CellPhoneDB [38] and CellTalkDB [39], typically focuses on individual species, such as humans or mice. The study of the ligand-receptor interaction across species has been an unexplored area. To this end, the shared ligand-receptor (LR) pairs between humans and mice were confirmed in the CellTalkDB. Thereafter, these shared LR pairs were transformed using a human and mouse gene homology database derived from the Mouse Genome Informatics (MGI) database. These pairs were replaced with two different types of pairs: One contains human ligands and mouse receptors, and the other contains mouse ligands and human receptors. Finally, the co-localization of these LR pairs was evaluated using the STopover in scanpy, Python, based on normalized gene expression.

Analyzing cell-cell interactions between different biological systems, such as mouse and human cells, may be meaningful for two reasons: First, since the biological systems are relatively clearly distinct, the cell-cell interactions between different systems may be more easily identified, which is particularly important in cancer-immune, host-microbiome, and host-pathogen interactions. In addition, unique forms of cell-cell interactions, such as interspecies interactions, may lead to novel discoveries regarding isoforms and biomarkers. Several algorithms have been developed to analyze the cell-cell interactions, including CCCExplorer, CellChat, ICELLNET, and CellPhoneDB [14]. However, many of these algorithms have limitations due to their neglect of spatial proximity. However, recent studies have attempted to address this issue by applying machine learning algorithms to ST data [15]. Following this effort, we also introduced the STopover, an algorithm that utilizes topological analysis techniques to assess the joint abundance of two variables (e.g., cell type score, gene expression, or %human) in ST data [16].

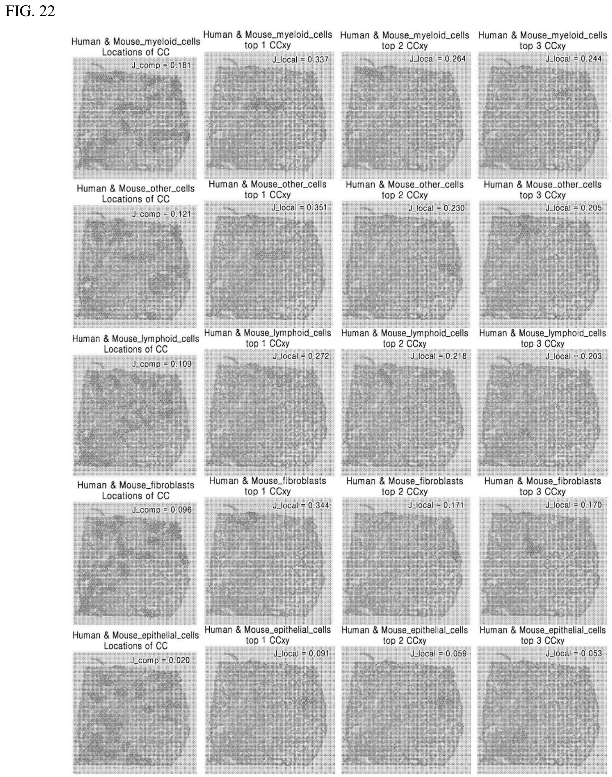

When performing the STopover, the endothelial cells showed the highest co-localization with %human among the six cell types (Jaccard index = 0.304) (FIGS. 21 and 22). This results match the previous Spearman correlation analysis performed based on CellDART (FIG. 20B). In addition, the ligand-receptor (LR) interactions between different species using STopover and CellTalkDB were explored. The CellTalkDB was modified for this study to analyze the interspecies interactions. As a result, the COL1A2 (human) and Cd93 (mouse) pair showed the highest Jaccard index, indicating the greatest spatial co-localization of this LR pair. Interestingly, Cd93 is a well-known marker gene for endothelial cells [17, 18]. This suggests spatial co-localization and potential interaction between mouse endothelial cells and human stem cells. Therefore, the STopover results confirm that the human stem cells are spatially aligned with mouse endothelial cells.

The comparative cell type analysis between 'Nor', 'Con', and 'Exp' observed changes in cell types due to lung fibrosis.

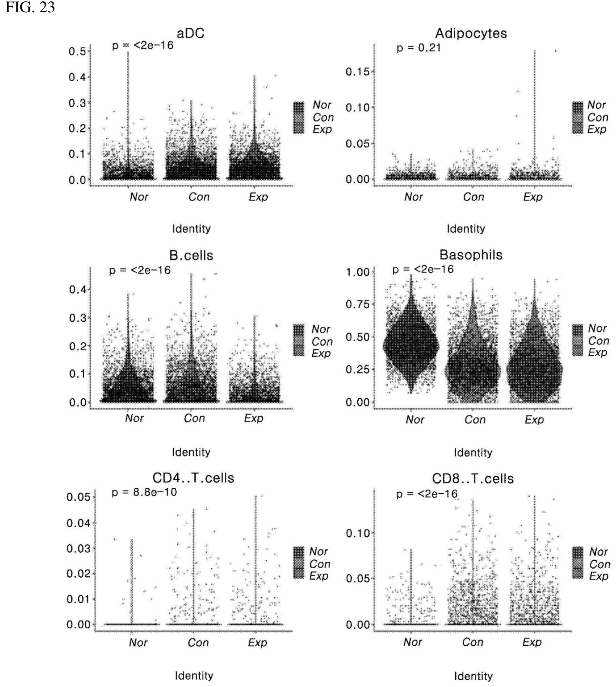

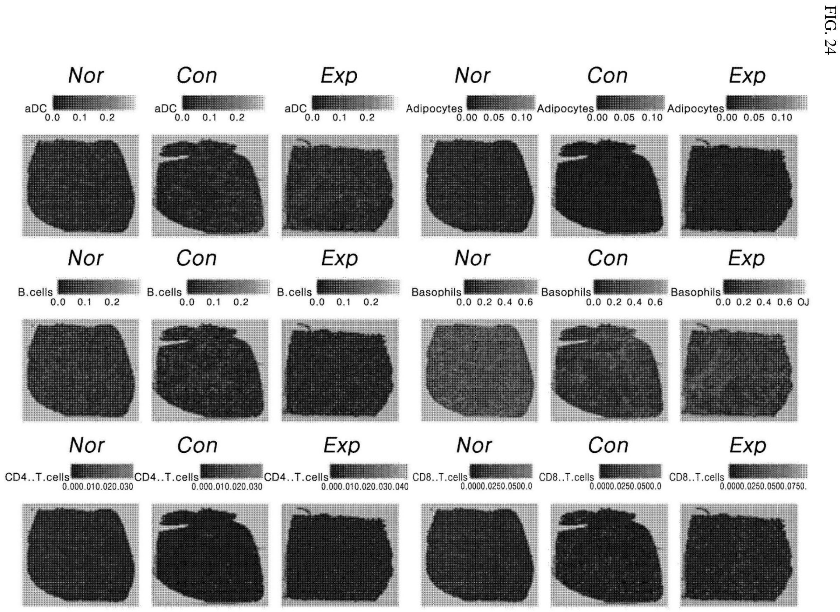

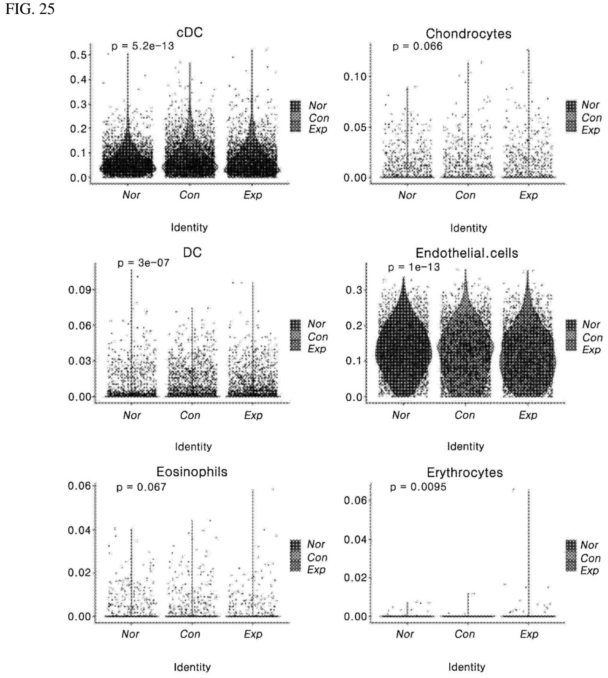

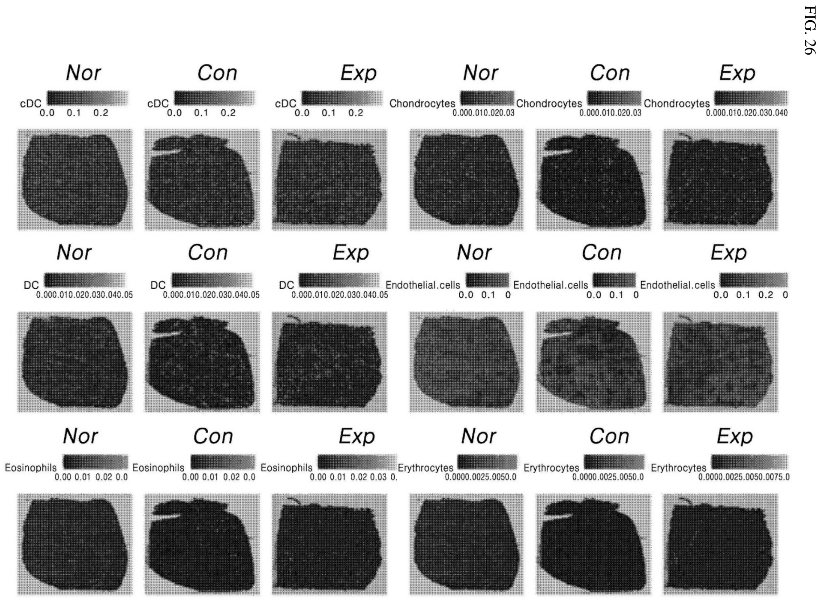

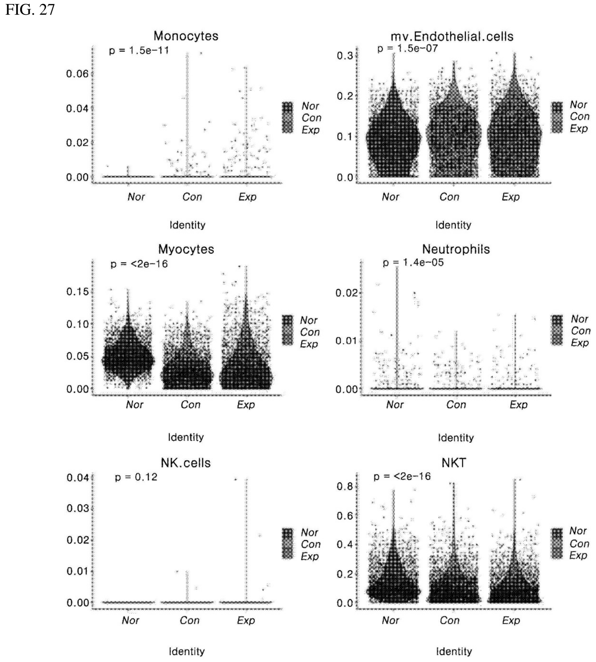

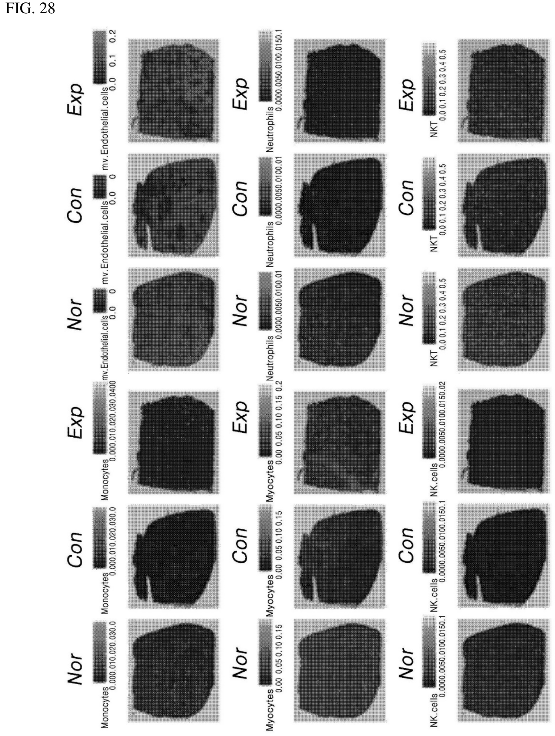

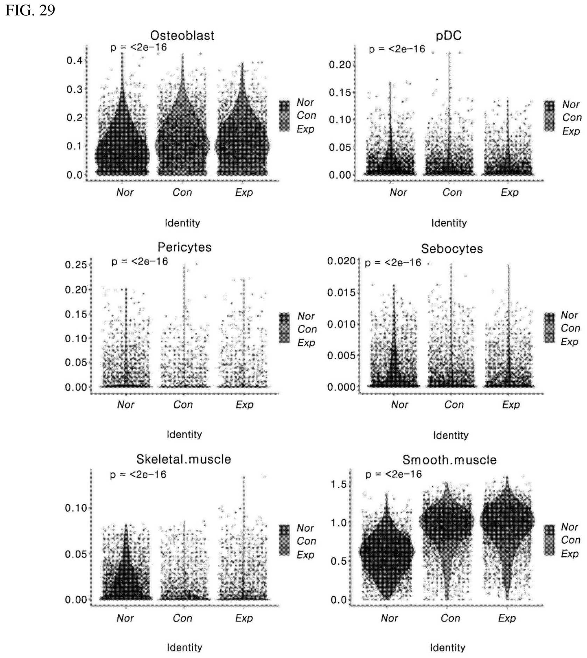

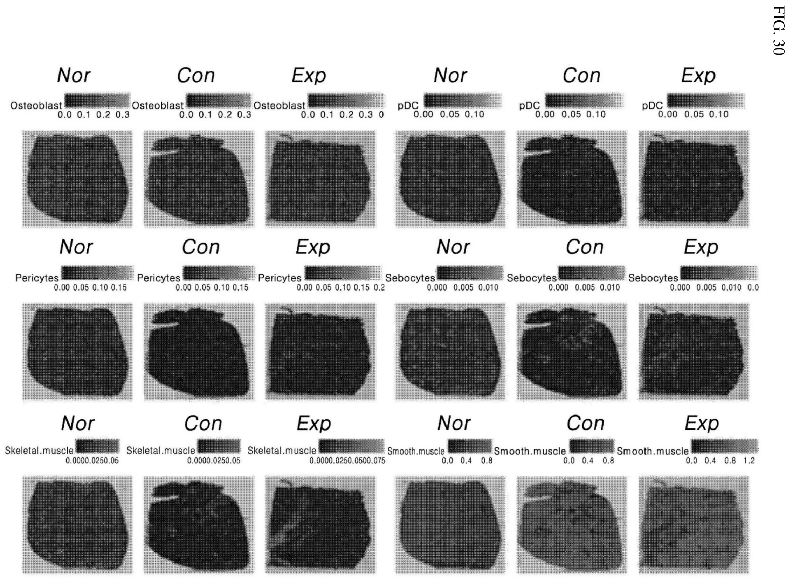

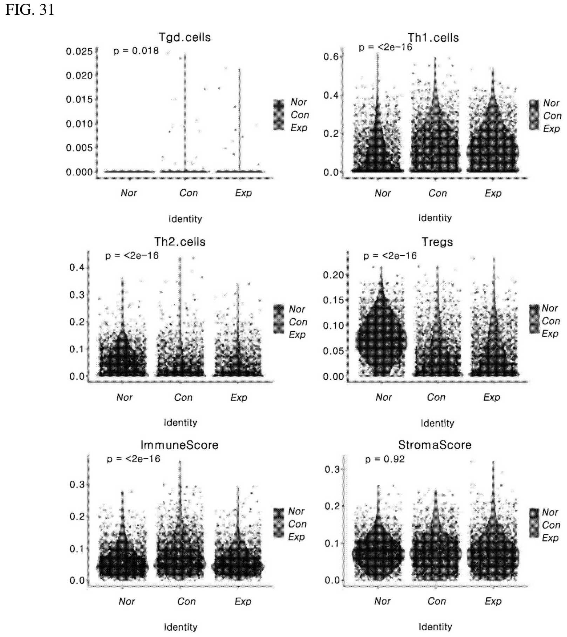

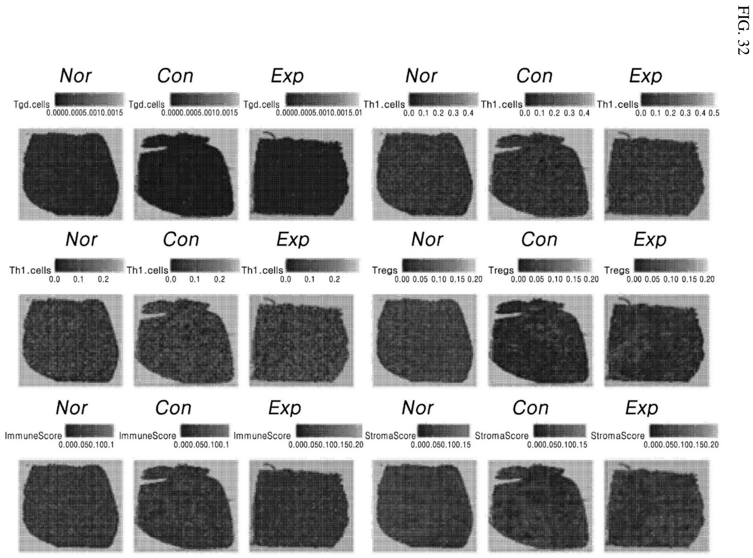

For the three samples (Nor, 'Con', and 'Exp'), various mouse cell types were identified, including aDCs, adipocytes, B cells, basophils, CD4 T cells, and CD8 T cells (FIGS. 23 and 24); cDC, chondrocytes, DC, endothelial cells, eosinophils, and erythrocytes (FIGS. 25 and 26); monocytes, mv. endothelial cells, myocytes, neutrophils, NK cells, and NKT (FIGS. 27 and 28); osteoblasts, pDCs, pericytes, sebocytes, skeletal muscle, and smooth muscle (FIGS. 29 and 30). CellDART was performed on Tgd cells (Th1 cells, Th2 cells, Tregs, Immune Score, and StromaScore) (FIGS. 31 and 32), and the results were shown in the figures above.

Comparison of the three samples observed the following changes in 'Con' compared to 'Nor':

[Table 1] [204]

| Cell type | Change | Description |
| --- | --- | --- |
| aDC | Increase | Implying increased activation of phagocytes due to pulmonary fibrosis |

(continued)

| Cell type | Change | Description |
|---|---|---|
| Basophils | Decrease | Implying decreased endothelial cells and reduced amount of granulocytes due to pulmonary fibrosis |
| Ly. Endothelial.cells | Decrease | Implying decreased endothelial cells and reduced amount of granulocytes due to pulmonary fibrosis |
| Macrophages | Increase | Implying increased activation of phagocytes due to pulmonary fibrosis |
| Macrophages. M1 | Increase | Implying increased activation of phagocytes due to pulmonary fibrosis |
| Macrophages. M2 | Increase | Implying increased activation of phagocytes due to pulmonary fibrosis |
| Myocytes | Decrease | Instead, implying increased stromal cells due to pulmonary fibrosis |
| Sebocytes | Decrease | Instead, implying increased stromal cells due to pulmonary fibrosis |
| Skeletal. muscle | Decrease | Instead, implying increased stromal cells due to pulmonary fibrosis |
| Smooth. muscle | Increase | Implying increased stromal cells due to pulmonary fibrosis |
| Th2.cells | Decrease | Implying increased phagocytes but decreased adaptive immunity |
| Tregs | Decrease | Implying increased phagocytes but decreased adaptive immunity |
| ImmuneScore | Increase | Implying increased activation of phagocytes due to pulmonary fibrosis |
| Comparison of DEGs between 'Nor', 'Con', and 'Exp' <br> 'Nor' vs 'Con' | | |

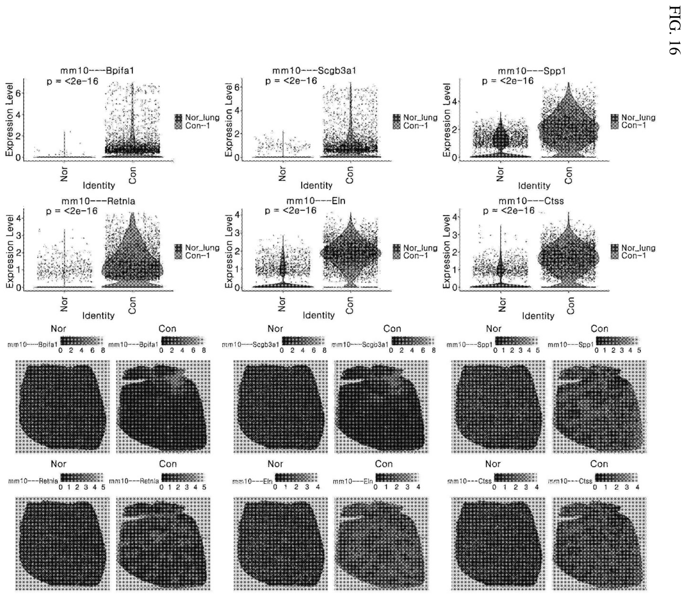

The DEGs of 'Nor' and 'Con' were confirmed using a volcano plot. Log FC > 0 indicates genes with higher expression in 'Con', and Log FC < 0 indicates genes with higher expression in 'Nor'. As a result, increased expression of extracellular matrix (ECM)-related genes was observed in 'Con' (FIGS. 14A, and C and D). In addition, comparing the top six differentially expressed genes (DEGs) in 'Nor' to 'Con' revealed that blood flow-related genes (e.g., Hba-a1, Hba-a2, Hbb-bs, and Hbb-bt) were present in 'Nor' (FIGS. 15 and 16). This is estimated to be evidence that blood vessels are deficient in the "Con" group due to fibrosis.

'Con' vs 'Exp'

The DEGs in 'Con' and 'Exp' were compared using a volcano plot. Log FC > 0 indicates genes with higher expression in 'Exp', and Log FC < 0 indicates genes with higher expression in 'Con'. As a result, blood flow activity was observed in 'Exp', suggesting a recovery mechanism (FIGS. 14B and E to F). In addition, blood flow-related genes (e.g., Hba-a1, Hba-a2, Hbb-bs, and Hbb-bt) reappeared in the 'Exp' sample (FIGS. 14B, E to F, 17, and 18). This suggests that the injected hMSCs exert a therapeutic effect on pulmonary fibrosis, at least at the molecular level, by restoring blood flow or blood vessels.

'Nor' vs 'Exp'

DEGs from 'Nor' and 'Exp' were compared using a volcano plot. Log FC > 0 indicates genes with higher expression in 'Exp', while Log FC < 0 indicates genes with higher expression in 'Nor'. As a result, the increase in ECM expression was observed in 'Exp' (FIGS. 29A and 29B).

Comparisons between the samples confirmed enhanced levels of ECM and immune responses in pulmonary fibrosis.

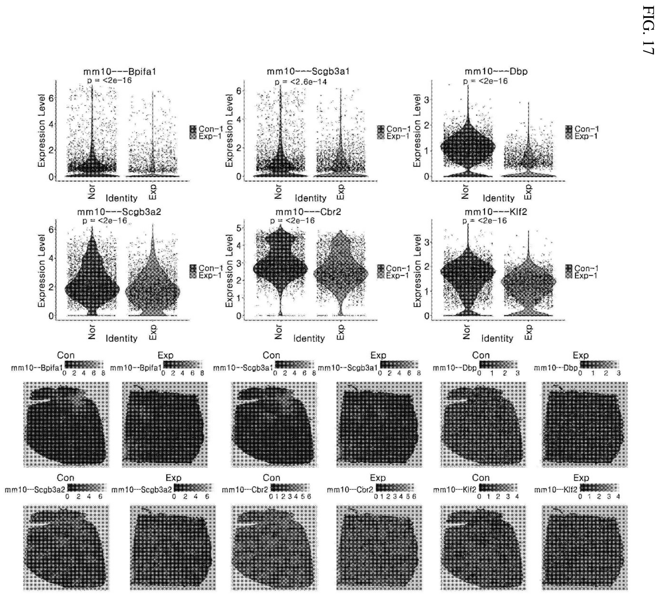

In addition, Bpifa1, a commonly upregulated DEG in 'Con' compared to 'Nor' or 'Exp', is known to be associated with innate immunity in lung infection models 30 (see FIGS. 16 and 17). Bpifa1 is also known to be associated with the mucosal environment of lung tissue. The presence of 'humoral immune response' as a primary GO term in spatially enriched genes in spatial cluster 5 (see FIG. 13D) and the enrichment of specific genes in 'Con' compared to 'Nor' or 'Exp' (see FIGS. 14D and E) may be related to Bpifa1. Therefore, decreased expression of this gene may be associated with therapeutic effect of injected human stem cells.

In addition, compared to 'Nor', Spp1, found in 'Con', is well known to be expressed in adjacent, invasive, or angiogenesis-associated macrophages 40. This also suggests a possible connection with innate immunity, which is related to the efficacy of hMSCs.

These results revealed three key findings.

First, we were able to distinguish RNA transcriptomes derived from different genomes and obtain their spatial distributions (FIG. 10).

Second, we were able to identify RNA transcriptomes of interest, i.e., genes significantly associated with hMSCs, through spatial clustering analysis (FIG. 13), differentially expressed genes (DEG) analysis (FIG. 14), and genes spatially associated with %human (FIG. 19).

Finally, the injected human stem cells showed a strong association with endothelial cells, while an inverse association with epithelial cells was observed.

The market for therapeutics containing nucleic acids is expanding along with numerous drugs currently under development. Clinical trials have been conducted on stem cells [19], CAR-T cells [20], and exosomes containing nucleic acids [21]. However, preclinical studies have reported difficulties in assessing the molecular mechanisms of these drugs, particularly their interactions with target tissue cells. As a result, there is a pressing need for methods for evaluating these drugs.

The ST-based method for identifying molecular markers spatially associated with injected drugs was previously developed [10]. This method identified the enhanced permeability and retention (EPR)-related markers and may be applied to various fluorescent dye-labeled therapeutics. However, labeling therapeutics with dyes has several drawbacks, including the potential for fluorescent dye labeling failure, altered drug properties, and dye dissociation or degradation [22-24]. This also applies to widely used methods for evaluating cell therapeutic agents using tracer labels. Herein we introduced a label-free approach to spatial mapping of exogenous nucleic acids using spatial transcriptomes. Previous studies have attempted to map RNA transcriptomes of different species in applications such as xenotransplantation [25], host-microbiome mapping [26], host-virus mapping [27], and engineered oligonucleotides [28]. While these studies demonstrated successful analysis of mixed transcriptomes, they did not utilize the distinct mapping technology of the present disclosure to identify the spatial distribution of cell therapeutic agents in tissues or infer their mode of action.

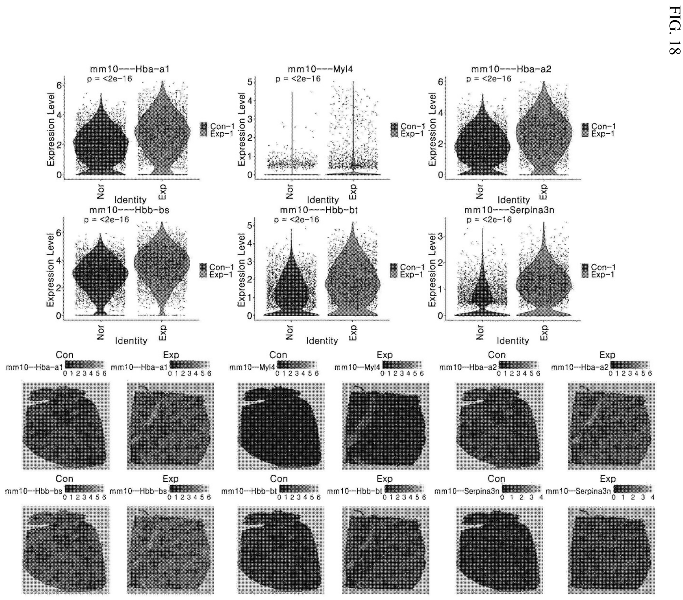

Furthermore, the present method may be applied to the cell therapeutic agents of the same origin as the host when transgenic genes are introduced that are not present in the host [29]. The approach proposed herein has the potential to extend the application of ST to the spatial analysis of therapeutic agents containing exogenous nucleic acids. This approach may obtain a more comprehensive understanding of the fundamental mechanisms underlying the therapeutic effects of these agents, which may lead to important insights for optimizing cell therapy treatments for various diseases. In addition to the simple distribution analysis, the spatial transcriptome analysis of stem cell therapy in the present disclosure may elucidate transcriptome-level effects on pulmonary fibrotic tissue. The injected human stem cells showed upregulation of several genes in the hemoglobin gene family, including Hba-a1, Hba-a2, Hbb-bs, and Hbb-bt, which were upregulated in the 'Exp' group compared to the 'Con' group (FIGS. 14 and 18). In addition, the 'Exp' group also showed downregulation of 'collagen-containing extracellular matrix' and 'humoral immune response' genes (e.g., Bpifal [30]) compared to the 'Con' group (FIGS. 13, 14, and 17). The molecular changes observed in the 'Exp' group were similar to those identified when the 'Nor' group was compared to the 'Con' group (e.g., Hba-a1, Hba-a2, Hbb-bs, and Hbb-bt) (FIGS. 15 and 16), thereby supporting the hypothesis that the molecular changes were due to the influence of the injected human stem cells.

Several issues should be considered when using the ST for spatial analysis of administered cells. The analysis presented herein relied on false-positive human transcriptomes, which were less significant in 'Nor' and 'Con'. The occurrence of false-positive human transcriptomes stems from homology between humans and mice. The false-negative human transcriptomes also need to be addressed. In the 'Exp' sample, VIM and Vim appeared among the top 20 genes spatially associated with %human (FIG. 19A). Given that VIM is associated with mesenchymal stem cells [31, 32], it is highly likely that Vim levels represent misclassified mouse transcriptomes. A potential solution to address the false detection problem presented herein is to employ long-read sequencing to fully manipulate gene sequence differences. Another potential approach is to develop computational algorithms that may reliably remove or correct false transcriptomes by manipulating spatial proximity [33], histological morphology [34, 35], previous datasets [12, 36, 37], or other domain knowledge.

The present disclosure successfully demonstrated the ability of ST to map administered cell therapeutic agents without fluorescent labeling. Key results demonstrated the spatial distribution of injected human stem cells in pulmonary fibrotic tissue and identified genes associated with this distribution.

Overall, the present disclosure demonstrates the utility of ST for spatial analysis of therapeutic agents containing exogenous nucleic acids.

Statistics

R (ver. 4.0.5) and Python (ver. 3.7.12) were used as programming languages. In addition, Seurat (ver. 4.0.2), scanpy (ver. 1.9.1), and SpaceRanger (ver. 2.0.1) were also used. For reference, SpaceRanger was applied to GRCh38 (Homo sapiens) and mm10 (Mus musculus). Differentially expressed genes (DEGs) were identified by classifying all genes with an adjusted p-value of less than 0.05 according to their fold change (FC) values. When plotting the gene ontology (GO) plot, 20 host mouse genes were selected and analyzed unless otherwise specified. Parameters were set to default values in Table 2 unless otherwise specified.

**Table 2 Specific Parameter value for Analysis**

| Function | Parametar | values |
|---|---|---|
| **Seurat in R** | | |
| **FindIntegrationAnchors** | **dims** | **1:50** |
| **IntegrateData** | **dims** | **1:50** |
| **EirrdfNeighibhors** | **dims** | **1:30** |
| **FindClusters** | **resolution** | **0.2** |
| **RunUMAP** | **dims** | **1:30** |
| *scanpy in Python* | | |
| *scanpy.ll.pca* | *svt-solver* | arpack |
| *scanpy.pp.neighbors* | *n_neiuhbors* | 10 |
| *scanpy.pp.neighbors* | *n_pcs* | 40 |
| *scanpy.ll.leiden* | *resolution* | 0.5 |
| *scanpy.pl.rank_genes groups* | *n_genes* | 40 |

Abbreviations

BBB: blood-brain barrier; BP: biological process; CAR-T/NK: chimeric antigen receptor-T cell or NK cell therapy; CC (GO analysis): cellular component; CC (STopover): connected component; CellDART: cell type inference by domain adaptation of single-cell and spatial transcriptome data; DEG: differentially expressed gene; ECM: extracellular matrix; FC: fold change; GO: gene ontology; hMSC: human mesenchymal stem cell; LR: ligand and receptor; MGI: mouse genome informatics; MOA: mechanism of action; OCT: optimal cutting temperature; PCR: polymerase chain reaction; qPCR: quantitative polymerase chain reaction; scRNA-seq: single-cell RNA sequencing; ST: spatially resolved transcriptome; TMM: trimmed mean of M-values; TO: tissue optimization

1. Saez-Ibanez AR, Upadhaya S, Partridge T, Shah M, Correa D, Campbell J. Landscape of cancer cell therapies: trends and real-world data. Nat Rev Drug Discov. 2022; 21: 631-2.

2. Ghobadinezhad F, Ebrahimi N, Mozaffari F, Moradi N, Beiranvand S, Pournazari M, et al. The emerging role of regulatory cell-based therapy in autoimmune disease. Front Immunol. 2022; 13: 1075813.

3. Hossein-Khannazer N, Torabi S, Hosseinzadeh R, Shahrokh S, Asadzadeh Aghdaei H, Memarnejadian A, et al. Novel cell-based therapies in inflammatory bowel diseases: the established concept, promising results. Hum Cell. 2021; 34: 1289-300.

4. Pradhan AU, Uwishema O, Onyeaka H, Adanur I, Dost B. A review of stem cell therapy: An emerging treatment for dementia in Alzheimer's and Parkinson's disease. Brain Behav. 2022; 12: e2740.

5. Xia Y, Rao L, Yao H, Wang Z, Ning P, Chen X. Engineering Macrophages for Cancer Immunotherapy and Drug Delivery. Adv Mater. 2020; 32: e2002054.

6. Zhuang WZ, Lin YH, Su LJ, Wu MS, Jeng HY, Chang HC, et al. Mesenchymal stem/stromal cell-based therapy: mechanism, systemic safety and biodistribution for precision clinical applications. J Biomed Sci. 2021; 28: 28.

7. Brooks A, Futrega K, Liang X, Hu X, Liu X, Crawford DHG, et al. Concise Review: Quantitative Detection and Modeling the In Vivo Kinetics of Therapeutic Mesenchymal Stem/Stromal Cells. Stem Cells Transl Med. 2018; 7: 78-86.

8. Kreyling WG, Abdelmonem AM, Ali Z, Alves F, Geiser M, Haberl N, et al. In vivo integrity of polymer-coated gold nanoparticles. Nat Nanotechnol. 2015; 10: 619-23.

9. Bae S, Choi H, Lee DS. Discovery of molecular features underlying the morphological landscape by integrating spatial transcriptomic data with deep features of tissue images. Nucleic Acids Res. 2021; 49: e55.

10. Park J, Choi J, Lee JE, Choi H, Im HJ. Spatial Transcriptomics-Based Identification of Molecular Markers for Nanomedicine Distribution in Tumor Tissue. Small Methods. 2022; 6: e2201091.

11. Robinson MD, Oshlack A. A scaling normalization method for differential expression analysis of RNA-seq data.

Genome Biol. 2010; 11: R25.

12. Bae S, Na KJ, Koh J, Lee DS, Choi H, Kim YT. CellDART: cell type inference by domain adaptation of single-cell and spatial transcriptomic data. Nucleic Acids Res. 2022; 50: e57.

13. Jung KO, Kim TJ, Yu JH, Rhee S, Zhao W, Ha B, et al. Whole-body tracking of single cells via positron emission tomography. Nat Biomed Eng. 2020; 4: 835-44.

14. Armingol E, Officer A, Harismendy O, Lewis NE. Deciphering cell-cell interactions and communication from gene expression. Nat Rev Genet. 2021; 22: 71-88.

15. Fischer DS, Schaar AC, Theis FJ. Modeling intercellular communication in tissues using spatial graphs of cells. Nat Biotechnol. 2023; 41: 332-6.

16. Bae S, Lee H, Na KJ, Lee DS, Choi H, Kim YT. STopover captures spatial co-localization and interaction in the tumor microenvironment using topological analysis in spatial transcriptomics data. bioRxiv. 2022: 2022.11. 16.516708.

17. Ianevski A, Giri AK, Aittokallio T. Fully-automated and ultra-fast cell-type identification using specific marker combinations from single-cell transcriptomic data. Nat Commun. 2022; 13: 1246.

18. De Falco A, Caruso F, Su XD, Iavarone A, Ceccarelli M. A variational algorithm to detect the clonal copy number substructure of tumors from scRNA-seq data. Nat Commun. 2023; 14: 1074.

19. Ji Y, Hu C, Chen Z, Li Y, Dai J, Zhang J, et al. Clinical trials of stem cell-based therapies for pediatric diseases: a comprehensive analysis of trials registered on ClinicalTrials.gov and the ICTRP portal site. Stem Cell Res Ther. 2022; 13: 307.

20. Ivica NA, Young CM. Tracking the CAR-T Revolution: Analysis of Clinical Trials of CAR-T and TCR-T Therapies for the Treatment of Cancer (1997-2020). Healthcare (Basel). 2021; 9.

21. Zhang Y, Liu Q, Zhang X, Huang H, Tang S, Chai Y, et al. Recent advances in exosome-mediated nucleic acid delivery for cancer therapy. J Nanobiotechnology. 2022; 20: 279.

22. Srivastava AK, Bulte JW. Seeing stem cells at work in vivo. Stem cell reviews and reports. 2014; 10: 127-44.

23. Horan PK, Melnicoff MJ, Jensen BD, Slezak SE. Fluorescent cell labeling for in vivo and in vitro cell tracking. Methods Cell Biol. 1990; 33: 469-90.

24. Choi H, Lee DS. Illuminating the physiology of extracellular vesicles. Stem Cell Res Ther. 2016; 7: 55.

25. Ni Z, Prasad A, Chen S, Halberg RB, Arkin LM, Drolet BA, et al. SpotClean adjusts for spot swapping in spatial transcriptomics data. Nat Commun. 2022; 13: 2971.

26. Galeano Nino JL, Wu H, LaCourse KD, Kempchinsky AG, Baryiames A, Barber B, et al. Effect of the intratumoral microbiota on spatial and cellular heterogeneity in cancer. Nature. 2022; 611: 810-7.

27. Sounart H, Lazar E, Masarapu Y, Wu J, Varkonyi T, Glasz T, et al. Dual spatially resolved transcriptomics for SARS-CoV-2 host-pathogen co-localization studies in humans. bioRxiv. 2022: 2022.03. 14.484288.

28. Ben-Chetrit N, Niu X, Swett AD, Sotelo J, Jiao MS, Stewart CM, et al. Integration of whole transcriptome spatial profiling with protein markers. Nat Biotechnol. 2023.

29. 10x Genomics. Build a Custom Reference. Retrieved from https://support.10xgenomics.com/single-cell-gen e-expression/software/pipelines/latest/using/tutorial_mr.

30. Tsou YA, Tung MC, Alexander KA, Chang WD, Tsai MH, Chen HL, et al. The Role of BPIFA1 in Upper Airway Microbial Infections and Correlated Diseases. Biomed Res Int. 2018; 2018: 2021890.

31. Zhang L, Wei Y, Chi Y, Liu D, Yang S, Han Z, et al. Two-step generation of mesenchymal stem/stromal cells from human pluripotent stem cells with reinforced efficacy upon osteoarthritis rabbits by HA hydrogel. Cell Biosci. 2021; 11: 6.

32. Cooper TT, Sherman SE, Bell GI, Ma J, Kuljanin M, Jose SE, et al. Characterization of a Vimentin(high) /Nestin(high) proteome and tissue regenerative secretome generated by human pancreas-derived mesenchymal stromal cells. Stem Cells. 2020; 38: 666-82.

33. Long Y, Ang KS, Li M, Chong KLK, Sethi R, Zhong C, et al. Spatially informed clustering, integration, and deconvolution of spatial transcriptomics with GraphST. Nat Commun. 2023; 14: 1155.

34. Comiter C, Vaishnav ED, Ciapmricotti M, Li B, Yang Y, Rodig SJ, et al. Inference of single cell profiles from histology stains with the Single-Cell omics from Histology Analysis Framework (SCHAF). bioRxiv. 2023: 2023.03. 21.533680.

35. Parreno-Centeno M, Malagoli Tagliazucchi G, Withnell E, Pan S, Secrier M. A deep learning and graph-based approach to characterise the immunological landscape and spatial architecture of colon cancer tissue. bioRxiv. 2022: 2022.07. 06.498984.

36. Li H, Zhou J, Li Z, Chen S, Liao X, Zhang B, et al. A comprehensive benchmarking with practical guidelines for cellular deconvolution of spatial transcriptomics. Nat Commun. 2023; 14: 1548.

37. Miller BF, Huang F, Atta L, Sahoo A, Fan J. Reference-free cell type deconvolution of multi-cellular pixel-resolution spatially resolved transcriptomics data. Nat Commun. 2022; 13: 2339.

38. Efremova M, Vento-Tormo M, Teichmann SA, Vento-Tormo R. CellPhoneDB: inferring cell-cell communication from combined expression of multi-subunit ligand-receptor complexes. Nat Protoc. 2020; 15: 1484-506.

39. Shao X, Liao J, Li C, Lu X, Cheng J, Fan X. CellTalkDB: a manually curated database of ligand-receptor interactions in humans and mice. Brief Bioinform. 2021; 22.
40. Cheng, S. et al. A pan-cancer single-cell transcriptional atlas of tumor infiltrating myeloid cells. Cell 184, 792-809 e723 (2021).

**Claims**

1. A method for exploring distribution, efficacy, action, or physiological activity of a genome-containing material within a tissue of interest, comprising:

   obtaining spatially resolved transcriptome information of the tissue of interest by providing a genome-containing material of part or all of a genome not present in the tissue of interest to the tissue of interest that has or is induced by a disease or illness;
   classifying tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs; and
   exploring the distribution, mechanism, efficacy, action, or physiological activity of the genome-containing material within the tissue of interest from the classified information.

2. The method of claim 1, wherein the exploration includes extracting a molecular marker spatially associated with a ratio of the number of genome-derived transcriptomes to the total number of spatial transcriptomes within the tissue of interest.

3. The method of claim 1, wherein each origin information is tagged to spatially resolved transcriptome information of the tissue of interest by the classification.

4. The method of claim 1, wherein the exploration includes segmenting the spatially resolved transcriptome image of the classified information or the entire spatially resolved transcriptome image into one or more clusters.

5. The method of claim 4, wherein an analysis is performed on one or more clusters using an image feature extraction algorithm for individual clusters, a correlation analysis between image intensity of a cluster and a gene expression level, a cell type analysis algorithm, and/or a gene ontology analysis.

6. The method of claim 1, wherein the exploration includes performing a cell type analysis algorithm or a gene ontology (GO) analysis on the spatially resolved transcriptome information of the classified information or the entire spatially resolved transcriptome information.

7. The method of claim 6, wherein the cell type analysis algorithm is a Fisher's exact test, maximum likelihood estimation, domain adaptive classification, logistic regression analysis, or negative binomial regression analysis algorithm.

8. The method of claim 6, wherein the cell type analysis algorithm is CellDART algorithm, spSeudoMap algorithm, xCell algorithm, STopover algorithm, RCTD algorithm, Seurat algorithm, celltypist algorithm, cell2location algorithm, Scanorama algorithm, SPOTlight algorithm, DSTG algorithm, CellTrek algorithm, sc-type algorithm, TIMER algorithm, CIBERSORT algorithm, MCP-counter algorithm, Quantiseq algorithm, EPIC algorithm, or scanpy's injest algorithm.

9. The method of claim 1, further comprising:
   obtaining comparative transcriptome information of a second tissue of interest that has or is caused by the same disease or illness as the tissue of interest without providing the genome-containing material, and using the comparative transcriptome information in the exploration.

10. The method of claim 9, comprising:
    performing a correlation analysis between a gene expression level based on the comparative transcriptome information and a gene expression level based on the spatially resolved transcriptome information.

11. The method of claim 10, wherein the correlation analysis utilizes differentially expressed genes (DEGs), a correlation analysis based on a correlation coefficient calculation, or an image similarity evaluation algorithm.

12. The method of claim 11, wherein the correlation coefficient calculation is a Pearson correlation coefficient, a Spearman correlation coefficient, or a Kendall correlation coefficient.

13. The method of claim 1, further comprising:
obtaining normal transcriptome information from a normal third tissue of interest that does not have or is not induced by the disease or illness and is not provided with the genome-containing material, and using the normal transcriptome information in the exploration.

14. The method of any one of claims 1 to 13, wherein the provision of the genome-containing material to the tissue of interest includes directly providing the material to the tissue of interest or systemically administering the material to a subject and then distributing the material to the tissue of interest.

15. The method of any one of claims 9 to 13, wherein the exploration further includes comparing one or more of the spatially resolved transcriptome information, comparative transcriptome information, and normal transcriptome information of the tissue of interest to identify a molecular marker derived from the genome-containing material that is enhanced or suppressed.

16. The method of any one of claims 1 to 13, further comprising:
staining at least one of the tissue of interest, the second tissue of interest, and the third tissue of interest, and obtaining a stained image of the tissue.

17. The method of claim 16, further comprising:
segmenting the stained image into one or more clusters, and analyzing the one or more clusters using the image feature extraction algorithm for individual clusters, the correlation analysis between the image intensity of the cluster and the gene expression level of the genome-containing material, the cell type analysis algorithm, and/or the gene ontology analysis.

18. The method of any one of claims 1 to 13, wherein the spatially resolved transcriptome information is the number and type of transcriptome RNA reads within a spot having spatial coordinates within the tissue of interest.

19. The method of any one of claims 1 to 13, wherein the genome-containing material is a cell, an extracellular vesicle, a lymphocyte, a microbiome, a vector, or a virus containing a genome.

20. The method of any one of claims 9 to 13, further comprising:
segmenting one or more tissue images of interest, the second tissue of interest, and the third tissue of interest into one or more clusters.

21. The method of claim 20, wherein the one or more clusters are classified by intensity of the image, or classified by an algorithm that segments a tissue of interest image into a plurality of patches and classifies the tissue of interest image by a similarity of image features for each patch.

22. A method for classifying genome-derived transcriptome information from spatially resolved transcriptome information, comprising:

obtaining spatially resolved transcriptome information of the tissue of interest by providing a genome-containing material of part or all of a genome not present in the tissue of interest to the tissue of interest that has or is induced by a disease or illness; and
classifying genome-derived transcriptome information among spatially resolved transcriptome information using an integrated reference transcriptome library including first organism reference transcriptome information to which the tissue of interest belongs and second organism reference transcriptome information to which the genome or a genome-containing material belongs.

23. The method of claim 22, wherein the method has higher classification accuracy compared to classifying genome-derived transcriptome information among spatially resolved transcriptome information using first or second organism reference transcriptome information, without using an integrated reference transcriptome library.

24. An apparatus (100) for exploring distribution, efficacy, action, or physiological activity of a genome-containing material within a tissue of interest, comprising:

an information receiving unit (110) that receives spatially resolved transcriptome information of the tissue of interest by providing a genome-containing material of part or all of a genome not present in the tissue of interest to the tissue of interest that has or is induced by a disease or illness;
an origin exploration unit (120) that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs; and
a molecular marker exploration unit (130) that explores a molecular marker related to the distribution, efficacy, action, or physiological activity of the genome-containing material within the tissue of interest from the information of the origin exploration unit (120).

25. The apparatus (100) of claim 24, wherein the molecular marker exploration unit (130) further includes extracting a molecular marker spatially associated with a ratio of the number of genome-derived transcriptomes to the total number of spatial transcriptomes of the tissue of interest from the information of the origin exploration unit (120).

26. The apparatus (100) of claim 24, wherein a comparative transcriptome information receiving unit (111) that obtains comparative transcriptome information of a second tissue of interest that has or is caused by the same disease or illness as the tissue of interest without providing the genome-containing material, and receives the obtained information.

27. The apparatus (100) of claim 24, wherein the molecular marker exploration unit (130) performs a correlation analysis between a gene expression level of the comparative transcriptome information receiving unit (111) and a gene expression level of the spatially resolved transcriptome information of the information receiving unit (110) or the origin exploration unit (120).

28. The apparatus (100) of claim 24, further comprising:
a normal transcriptome information receiving unit (112) that obtains normal transcriptome information of a normal third tissue of interest that does not have or is not induced by the disease or illness and is not provided with the genome-containing material, and receives the obtained information.

29. The apparatus (100) of any one of claims 24 to 28, wherein the molecular marker exploration unit (130) explores the molecular marker derived from the genome-containing material that are enhanced or suppressed by comparing the spatially resolved transcriptome information from one or more of the information receiving unit (110), the origin exploration unit (120), the comparative transcriptome information receiving unit (111), and the normal transcriptome information receiving unit (112).

30. The apparatus (100) of any one of claims 24 to 28, wherein the molecular marker exploration unit (130) includes at least one of an image feature analysis unit (131) that extracts transcriptome information using an image feature extraction algorithm using artificial intelligence, a correlation analysis unit (132) that extracts the transcriptome information by analyzing a correlation between image intensity and a gene expression level of a tissue, a cell type analysis unit (133) that uses a cell type analysis algorithm, a gene ontology analysis unit (134) that uses a gene ontology analysis, and a clustering unit (135).

31. The apparatus (100) of claim 30, wherein the cell type analysis unit (133) uses a Fisher's exact test, maximum likelihood estimation, domain adaptive classification, logistic regression analysis, or negative binomial regression analysis algorithm.

32. The apparatus (100) of claim 30, wherein the cell type analysis unit (130) analyzes a cell type using CellDART algorithm, spSeudoMap algorithm, xCell algorithm, STopover algorithm, RCTD algorithm, Seurat algorithm, celltypist algorithm, cell2location algorithm, Scanorama algorithm, SPOTlight algorithm, DSTG algorithm, CellTrek algorithm, sc-type algorithm, TIMER algorithm, CIBERSORT algorithm, MCP-counter algorithm, Quantiseq algorithm, EPIC algorithm, or scanpy's injest algorithm.

33. The apparatus (100) of claim 30, wherein the clustering unit (135) classifies one or more clusters by image intensity of the tissue of interest, or classifies a tissue of interest image into a plurality of patches and classifies the tissue of interest image by a similarity of image features for each patch.

34. An apparatus (200) for classifying genome-derived transcriptome information from spatially resolved transcriptome information, comprising:

an information receiving unit **(110)** that receives spatially resolved transcriptome information of the tissue of interest by providing a genome-containing material of part or all of a genome not present in the tissue of interest to the tissue of interest that has or is induced by a disease or illness; and
an origin exploration unit (120) that classifies tissue-of-interest-derived transcriptome information and genome-derived transcriptome information among spatially resolved transcriptome information by an integrated reference transcriptome library including first organism reference transcriptome information to which the tissue of interest belongs and second organism reference transcriptome information to which the genome or a genome-containing material belongs.

FIG. 1

ACQUIRE SPATIALLY RESOLVED
TRANSCRIPTOME INFORMATION OF
TISSUE OF INTEREST BY PROVIDING
GENOME-CONTAINING MATERIAL ⸺S10

CLASSIFY SPATIALLY RESOLVED
TRANSCRIPTOME INFORMATION FOR
EACH ORIGIN BY INTEGRATED
REFERENCE TRANSCRIPTOME LIBRARY ⸺S20

EXPLORE DISTRIBUTION, MECHANISM,
EFFICACY, ACTION, OR PHYSIOLOGICAL
ACTIVITY OF GENOME-CONTAINING
MATERIAL WITHIN TISSUE OF INTEREST
FROM CLASSIFIED INFORMATION ⸺S30

FIG. 2

ACQUIRE SPATIALLY RESOLVED TRANSCRIPTOME
INFORMATION OF TISSUE OF INTEREST (S10)

ESTABLISH
ANIMAL MODEL
FOR DISEASE
OF INTEREST

PROVIDE GENOME-
CONTAINING
MATERIAL TO TISSUE
OF INTEREST
IN ANIMAL MODEL
FOR DISEASE OF
INTEREST

ACQUIRE SPATIALLY
RESOLVED
TRANSCRIPTOME
INFORMATION OF
TISSUE OF INTEREST
THROUGH RNA
SEQUENCING, ETC.,
OF FROZEN SECTION
OF TISSUE OF
INTEREST

FIG. 3

FIG. 4

EP 4 696 771 A1

FIG. 5

EXPLORE PHYSIOLOGICAL ACTIVITY, ETC., OF
GENOME-CONTAINING MATERIAL (S30)

IMAGE FEATURE EXTRACTION ALGORITHM (ex. SPADE)

CORRELATION ANALYSIS BETWEEN IMAGE INTENSITY AND GENE EXPRESSION LEVEL OF TISSUE OF INTEREST (ex. DEG)

CELL TYPE ANALYSIS ALGORITHM (ex. CellDART, MIA, STopover, RCTD)

GENE ONTOLOGY ANALYSIS

FIG. 6

Nor

Con

Exp

hMSC

OCT Mold

H&E-Stained Tissue

Frozen Tissue for Visium

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

*a* Volcano plot
*EnhancedVolcano*

*b*

total = 828 variables

FIG.34

FIG.35

FIG.36

FIG.37

## INTERNATIONAL SEARCH REPORT

International application No.

**PCT/KR2023/011786**

### A.    CLASSIFICATION OF SUBJECT MATTER

**C12N 5/0775**(2010.01)i; **A61K 35/28**(2006.01)i; **A61P 11/00**(2006.01)i

According to International Patent Classification (IPC) or to both national classification and IPC

### B.    FIELDS SEARCHED

Minimum documentation searched (classification system followed by classification symbols)

C12N 5/0775(2010.01); G16B 15/30(2019.01); G16B 25/00(2019.01); G16B 40/20(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유전체(genome), 공간 전사체(spatial transcriptome), 분자적 마커(molecular marker), 클러스터(cluster), 온톨로지(ontology), 분류(classification)

### C.    DOCUMENTS CONSIDERED TO BE RELEVANT

| Category* | Citation of document, with indication, where appropriate, of the relevant passages | Relevant to claim No. |
|---|---|---|
| A | KR 10-2023-0000948 A (PORTRAI INC.) 03 January 2023 (2023-01-03)<br>    See abstract; and claims 1-22. | 1-13,15-34 |
| A | BAE, S. et al. Discovery of molecular features underlying the morphological landscape by integrating spatial transcriptomic data with deep features of tissue images. Nucleic Acids Research. 2021, vol. 49, no. 10, thesis no. e55, pp. 1-12.<br>    See abstract; and figures 1-4. | 1-13,15-34 |
| A | PARK, J. et al. Spatial transcriptomics-based identification of molecular markers for nanomedicine distribution in tumor tissue. Small Methods. 2022, thesis no. 2201091, pp. 1-13.<br>    See abstract; and figures 1-5. | 1-13,15-34 |
| A | FERREIRA, R. M. et al. Integration of spatial and single-cell transcriptomics localizes epithelial cell-immune cross-talk in kidney injury. JCI Insight. 2021, vol. 6, no. 12, thesis no. e147703, pp. 1-21.<br>    See entire document. | 1-13,15-34 |
| A | KR 10-2022-0142905 A (PORTRAI INC.) 24 October 2022 (2022-10-24)<br>    See entire document. | 1-13,15-34 |

☐ Further documents are listed in the continuation of Box C.          ☑ See patent family annex.

| | | |
|---|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family | |

| Date of the actual completion of the international search | Date of mailing of the international search report |
|---|---|
| **08 January 2024** | **09 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | |
|---|---|
| International application No. | |
| | **PCT/KR2023/011786** |

| **Box No. II** | **Observations where certain claims were found unsearchable (Continuation of item 2 of first sheet)** |
|---|---|

This international search report has not been established in respect of certain claims under Article 17(2)(a) for the following reasons:

1. ☑ Claims Nos.: **14**
   because they relate to subject matter not required to be searched by this Authority, namely:

   Claim 14 pertains to a method for treatment of the human body, including a step for direct treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

EP 4 696 771 A1

**INTERNATIONAL SEARCH REPORT**
Information on patent family members

International application No.

**PCT/KR2023/011786**

| Patent document cited in search report | | | Publication date (day/month/year) | Patent family member(s) | | | Publication date (day/month/year) |
|---|---|---|---|---|---|---|---|
| KR | 10-2023-0000948 | A | 03 January 2023 | KR | 10-2023-0000954 | A | 03 January 2023 |
| | | | | KR | 10-2489145 | B1 | 19 January 2023 |
| | | | | KR | 10-2489146 | B1 | 19 January 2023 |
| | | | | WO | 2022-270725 | A1 | 29 December 2022 |
| | | | | WO | 2022-270770 | A1 | 29 December 2022 |
| KR | 10-2022-0142905 | A | 24 October 2022 | AU | 2022-257481 | A1 | 20 October 2022 |
| | | | | WO | 2022-220385 | A1 | 20 October 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SAEZ-IBANEZ AR** ; **UPADHAYA S** ; **PARTRIDGE T** ; **SHAH M** ; **CORREA D** ; **CAMPBELL J**. Landscape of cancer cell therapies: trends and real-world data. *Nat Rev Drug Discov.*, 2022, vol. 21, 631-2

- **GHOBADINEZHAD F** ; **EBRAHIMI N** ; **MOZAFFARI F** ; **MORADI N** ; **BEIRANVAND S** ; **POURNAZARI M et al.** The emerging role of regulatory cell-based therapy in autoimmune disease. *Front Immunol.*, 2022, vol. 13, 1075813

- **HOSSEIN-KHANNAZER N** ; **TORABI S** ; **HOSSEIN-ZADEH R** ; **SHAHROKH S** ; **ASADZADEH AGH-DAEI H** ; **MEMARNEJADIAN A et al.** Novel cell-based therapies in inflammatory bowel diseases: the established concept, promising results. *Hum Cell.*, 2021, vol. 34, 1289-300

- **PRADHAN AU** ; **UWISHEMA O** ; **ONYEAKA H** ; **ADANUR I** ; **DOST B**. A review of stem cell therapy: An emerging treatment for dementia in Alzheimer's and Parkinson's disease. *Brain Behav.*, 2022, vol. 12, e2740

- **XIA Y** ; **RAO L** ; **YAO H** ; **WANG Z** ; **NING P** ; **CHEN X.** Engineering Macrophages for Cancer Immunotherapy and Drug Delivery. *Adv Mater.*, 2020, vol. 32, e2002054

- **ZHUANG WZ** ; **LIN YH** ; **SU LJ** ; **WU MS** ; **JENG HY** ; **CHANG HC et al.** Mesenchymal stem/stromal cell-based therapy: mechanism, systemic safety and biodistribution for precision clinical applications. *J Biomed Sci.*, 2021, vol. 28, 28

- **BROOKS A** ; **FUTREGA K** ; **LIANG X** ; **HU X** ; **LIU X** ; **CRAWFORD DHG et al.** Concise Review: Quantitative Detection and Modeling the In Vivo Kinetics of Therapeutic Mesenchymal Stem/Stromal Cells. *Stem Cells Transl Med.*, 2018, vol. 7, 78-86

- **KREYLING WG** ; **ABDELMONEM AM** ; **ALI Z** ; **ALVES F** ; **GEISER M** ; **HABERL N et al.** In vivo integrity of polymer-coated gold nanoparticles. *Nat Nanotechnol.*, 2015, vol. 10, 619-23

- **BAE S** ; **CHOI H** ; **LEE DS**. Discovery of molecular features underlying the morphological landscape by integrating spatial transcriptomic data with deep features of tissue images. *Nucleic Acids Res.*, 2021, vol. 49, e55

- **PARK J** ; **CHOI J** ; **LEE JE** ; **CHOI H** ; **IM HJ**. Spatial Transcriptomics-Based Identification of Molecular Markers for Nanomedicine Distribution in Tumor Tissue. *Small Methods*, 2022, vol. 6, e2201091

- **ROBINSON MD** ; **OSHLACK A**. A scaling normalization method for differential expression analysis of RNA-seq data. *Genome Biol.*, 2010, vol. 11, R25

- **BAE S** ; **NA KJ** ; **KOH J** ; **LEE DS** ; **CHOI H** ; **KIM YT**. CellDART: cell type inference by domain adaptation of single-cell and spatial transcriptomic data. *Nucleic Acids Res.*, 2022, vol. 50, e57

- **JUNG KO** ; **KIM TJ** ; **YU JH** ; **RHEE S** ; **ZHAO W** ; **HA B et al.** Whole-body tracking of single cells via positron emission tomography. *Nat Biomed Eng.*, 2020, vol. 4, 835-44

- **ARMINGOL E** ; **OFFICER A** ; **HARISMENDY O** ; **LEWIS NE**. Deciphering cell-cell interactions and communication from gene expression. *Nat Rev Genet.*, 2021, vol. 22, 71-88

- **FISCHER DS** ; **SCHAAR AC** ; **THEIS FJ**. Modeling intercellular communication in tissues using spatial graphs of cells. *Nat Biotechnol.*, 2023, vol. 41, 332-6

- **BAE S** ; **LEE H** ; **NA KJ** ; **LEE DS** ; **CHOI H** ; **KIM YT**. STopover captures spatial co-localization and interaction in the tumor microenvironment using topological analysis in spatial transcriptomics data. *bioRxiv*, 2022

- **IANEVSKI A** ; **GIRI AK** ; **AITTOKALLIO T**. Fully-automated and ultra-fast cell-type identification using specific marker combinations from single-cell transcriptomic data. *Nat Commun.*, 2022, vol. 13, 1246

- **DE FALCO A** ; **CARUSO F** ; **SU XD** ; **IAVARONE A** ; **CECCARELLI M**. A variational algorithm to detect the clonal copy number substructure of tumors from scRNA-seq data. *Nat Commun.*, 2023, vol. 14, 1074

- **JI Y** ; **HU C** ; **CHEN Z** ; **LI Y** ; **DAI J** ; **ZHANG J et al.** Clinical trials of stem cell-based therapies for pediatric diseases: a comprehensive analysis of trials registered on ClinicalTrials.gov and the ICTRP portal site. *Stem Cell Res Ther.*, 2022, vol. 13, 307

- **IVICA NA** ; **YOUNG CM**. Tracking the CAR-T Revolution: Analysis of Clinical Trials of CAR-T and TCR-T Therapies for the Treatment of Cancer (1997-2020). *Healthcare (Basel)*, 2021, 9

- **ZHANG Y** ; **LIU Q** ; **ZHANG X** ; **HUANG H** ; **TANG S** ; **CHAI Y et al.** Recent advances in exosome-mediated nucleic acid delivery for cancer therapy. *J Nanobiotechnology*, 2022, vol. 20, 279

- **SRIVASTAVA AK** ; **BULTE JW**. Seeing stem cells at work in vivo. *Stem cell reviews and reports*, 2014, vol. 10, 127-44
- **HORAN PK** ; **MELNICOFF MJ** ; **JENSEN BD** ; **SLEZAK SE**. Fluorescent cell labeling for in vivo and in vitro cell tracking. *Methods Cell Biol.*, 1990, vol. 33, 469-90
- **CHOI H** ; **LEE DS**. Illuminating the physiology of extracellular vesicles. *Stem Cell Res Ther.*, 2016, vol. 7, 55
- **NI Z** ; **PRASAD A** ; **CHEN S** ; **HALBERG RB** ; **ARKIN LM** ; **DROLET BA et al.** SpotClean adjusts for spot swapping in spatial transcriptomics data. *Nat Commun.*, 2022, vol. 13, 2971
- **GALEANO NINO JL** ; **WU H** ; **LACOURSE KD** ; **KEMPCHINSKY AG** ; **BARYIAMES A** ; **BARBER B et al.** Effect of the intratumoral microbiota on spatial and cellular heterogeneity in cancer. *Nature*, 2022, vol. 611, 810-7
- **SOUNART H** ; **LAZAR E** ; **MASARAPU Y** ; **WU J** ; **VARKONYI T** ; **GLASZ T et al.** Dual spatially resolved transcriptomics for SARS-CoV-2 host-pathogen co-localization studies in humans. *bioRxiv*, 2022
- **BEN-CHETRIT N** ; **NIU X** ; **SWETT AD** ; **SOTELO J** ; **JIAO MS** ; **STEWART CM et al.** Integration of whole transcriptome spatial profiling with protein markers. *Nat Biotechnol.*, 2023
- *10x Genomics. Build a Custom Reference*, https://support.10xgenomics.com/single-cell-gene-expression/software/pipelines/latest/using/tutorial_mr
- **TSOU YA** ; **TUNG MC** ; **ALEXANDER KA** ; **CHANG WD** ; **TSAI MH** ; **CHEN HL et al.** The Role of BPIFA1 in Upper Airway Microbial Infections and Correlated Diseases. *Biomed Res Int.*, 2018, vol. 2018, 2021890
- **ZHANG L** ; **WEI Y** ; **CHI Y** ; **LIU D** ; **YANG S** ; **HAN Z et al.** Two-step generation of mesenchymal stem/stromal cells from human pluripotent stem cells with reinforced efficacy upon osteoarthritis rabbits by HA hydrogel. *Cell Biosci.*, 2021, vol. 11, 6
- **COOPER TT** ; **SHERMAN SE** ; **BELL GI** ; **MA J** ; **KULJANIN M** ; **JOSE SE et al.** Characterization of a Vimentin(high) /Nestin(high) proteome and tissue regenerative secretome generated by human pancreas-derived mesenchymal stromal cells. *Stem Cells*, 2020, vol. 38, 666-82
- **LONG Y** ; **ANG KS** ; **LI M** ; **CHONG KLK** ; **SETHI R** ; **ZHONG C et al.** Spatially informed clustering, integration, and deconvolution of spatial transcriptomics with GraphST. *Nat Commun.*, 2023, vol. 14, 1155
- **COMITER C** ; **VAISHNAV ED** ; **CIAPMRICOTTI M** ; **LI B** ; **YANG Y** ; **RODIG SJ et al.** Inference of single cell profiles from histology stains with the Single-Cell omics from Histology Analysis Framework (SCHAF). *bioRxiv*, 2023
- **PARRENO-CENTENO M** ; **MALAGOLI TAGLIA-ZUCCHI G** ; **WITHNELL E** ; **PAN S** ; **SECRIER M**. A deep learning and graph-based approach to characterise the immunological landscape and spatial architecture of colon cancer tissue. *bioRxiv*, 2022
- **LI H** ; **ZHOU J** ; **LI Z** ; **CHEN S** ; **LIAO X** ; **ZHANG B et al.** A comprehensive benchmarking with practical guidelines for cellular deconvolution of spatial transcriptomics. *Nat Commun.*, 2023, vol. 14, 1548
- **MILLER BF** ; **HUANG F** ; **ATTA L** ; **SAHOO A** ; **FAN J**. Reference-free cell type deconvolution of multi-cellular pixel-resolution spatially resolved transcriptomics data. *Nat Commun.*, 2022, vol. 13, 2339
- **EFREMOVA M** ; **VENTO-TORMO M** ; **TEICHMANN SA** ; **VENTO-TORMO R**. CellPhoneDB: inferring cell-cell communication from combined expression of multi-subunit ligand-receptor complexes. *Nat Protoc.*, 2020, vol. 15, 1484-506
- **SHAO X** ; **LIAO J** ; **LI C** ; **LU X** ; **CHENG J** ; **FAN X**. CellTalkDB: a manually curated database of ligand-receptor interactions in humans and mice. *Brief Bioinform.*, 2021, 22
- **CHENG, S. et al.** A pan-cancer single-cell transcriptional atlas of tumor infiltrating myeloid cells. *Cell*, 2021, vol. 184, 792-809, e723